# FASCICULE DE BREVET EUROPEEN

(11) **EP 0 673 933 B1**
(45) Date de publication et mention de la délivrance du brevet: **08.11.2000**
(21) Numéro de dépôt: 95400613.6
(22) Date de dépôt: 21.03.1995
(51) Int. Cl.: C07D 277/68, C07D 263/58, C07D 417/12, C07D 413/12, C07D 417/06, C07D 413/06, A61K 31/42, A61K 31/425

(54) **Aminoalkyl benzoxazolinones et benzothiazolinones, leur procédé de préparation et les compositions pharmaceutiques qui les contiennent**
Aminoalkyl Benzoxazolinone und Benzothiazolinone, Verfahren zu deren Herstellung und sie enthaltende Zusammensetzungen
Aminoalkyl benzoxazolinones and benzothiazolinones process for their preparation and compositions containing them

(30) Priorité: 22.03.1994 FR 9403298
(43) Date de publication de la demande: 27.09.1995
(73) Titulaire: ADIR ET COMPAGNIE, 92415 Courbevoie Cédex (FR)
(72) Inventeur: Mouithys-Mickalad, Ange, F-59120 Loos (FR); Depreux, Patrick, F-59280 Armentieres (FR); Lesieur, Daniel, F-59147 Gondecourt (FR); Adam, Gérard, F-79600 Le Mesnil le Roi (FR); Caignard, Daniel-Henri, F-75015 Paris (FR); Renard, Pierre, F-78000 Versailles (FR); Rettori, Marie-Claire, F-92400 Courbevoie (FR)

(56) Documents cités:
- EP-A- 0 110 781
- EP-A- 0 478 446
- EP-A- 0 506 539

## Description

La présente invention concerne de nouvelles aminoalkyl benzoxazolinones et benzothiazolinones, leur procédé de préparation et les compositions pharmaceutiques qui les contiennent.

La demande de brevet EP 506539 décrit des dérivés N-[(acylamino) éthyl] benzoxazolinones en tant qu'agents modulant la synthèse de la mélatonine.

La demande de brevet EP 478446 décrit des dérivés (aminoalkyl)benzothiazolinones en tant qu'agents antipsychotiques, analgésiques et anxiolytiques.

La demanderesse a présentement découvert de nouvelles aminoalkyl benzoxazolinones et benzothiazolinones qui possèdent, de façon surprenante, une affinité pour les récepteurs sigma (σ) beaucoup plus intense que les dérivés de la demande EP 478 446.

La très haute affinité des dérivés de l'invention pour les récepteurs sigma σ, nettement supérieure à celle obtenue avec les dérivés de la demande EP 478446 les rend utilisables dans la prévention et le traitement des maladies liées à ce type de récepteurs à savoir les désordres psychotiques, la protection neuronale, les troubles de la mémoire, l'insuffisance circulatoire cérébrale du sujet âgé, la maladie d'Alzheimer, les maladies inflammatoires de type immunitaire telles que l'arthrite, l'arthrite aiguë ou chronique, les troubles du péristaltisme intestinal.

Par ailleurs la haute sélectivité des dérivés de la présente invention pour les récepteurs sigma, en particulier leur absence d'affinité pour les récepteurs D₂, permet leur utilisation en thérapeutique avec une sécurité accrue. En particulier les effets secondaires de type extrapyramidaux que l'on rencontre lors du traitement avec des produits à forte composante D₂, à laquelle ces effets sont imputés, ne se retrouvent pas avec les produits de l'invention qui sont en moyenne 100 à 1000 fois moins affins pour les récepteurs D₂ que les dérivés de la demande EP 478446. Au bilan les produits de la présente invention présentent un rapport de sélectivité (affinité récepteurs sigma) : (affinité récetpeurs D₂) de 10 000 à 100 000 fois supérieur à celui obtenu avec les dérivés de la demande EP 478446 ce qui améliore considérablement leur sécurité d'emploi.

Plus particulièrement, la présente invention concerne les composés de formule (I) : 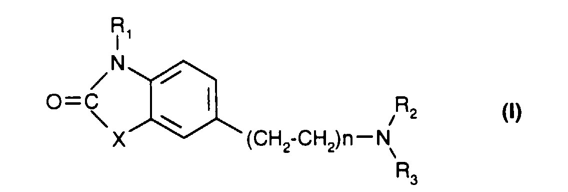 dans laquelle :
- R₁ représente un hydrogène ou un alkyle,
- n représente 1 ou 2,
- X représente un oxygène ou un soufre,
- R₂ représente un hydrogène ou un alkyle,
- et R₃ représente le groupement -(CH₂)ₘ-R₄ dans lequel :
   m représente 1, 2, 3 ou 4,
   et R₄ représente un groupement cycloalkylamino, dicycloalkylamino, N-cycloalkyl-N-alkylamino, ou un radical hétérocyclique de formule : 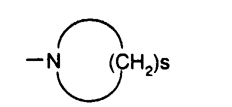 (avec s nombre entier compris entre 4 et 8 inclusivement), non substitué ou substitué par un groupement aryle, arylalkyle, aryle substitué, arylalkyl substitué,
- ou bien R₂ forme avec R₃ et l'atome d'azote qui les porte un groupement : 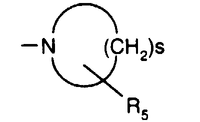 (avec s nombre entier compris entre 4 et 8 inclusivement) lequel groupement est substitué par un substituant R₅ choisi parmi aryle, arylalkyle, aryle substitué, et arylalkyle substitué,
- le terme "substitué" affectant les radicaux "aryle" et "arylalkyle" signifiant que ces groupements sont substitués par un, ou plusieurs groupements choisis parmi halogène, alkyle, hydroxyle, alkoxy, et trifluorométhyle,
- le terme "alkyle" et "alkoxy" désignent des groupements linéaires ou ramifiés contenant de 1 à 6 atomes de carbone,
- le terme "aryle" représente un groupement phényle ou naphtyle,
- le terme "cycloalkyle" désigne un groupement de 3 à 9 atomes de carbone,
leurs isomères optiques et leurs sels d'addition à un acide pharmaceutiquement acceptable et, lorsque R₁ représente un atome d'hydrogène, à une base pharmaceutiquement acceptable.

L'invention concerne particulièrement les composés de formule (I) dans lesquels X représente un soufre.

L'invention concerne particulièrement les composés dans lesquels R₂ forme avec R₃ et l'atome d'azote qui les porte un groupement : 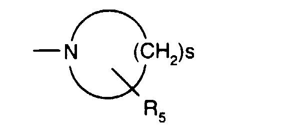 avec R₅ et s tels que définis dans la formule (I).

Parmi les acides pharmaceutiquement acceptables que l'on peut utiliser pour former un sel d'addition avec les composés de l'invention, on peut citer à titre d'exemples et de façon non limitative, les acides chlorhydrique, sulfurique, phosphorique, tartrique, malique, maléique, fumarique, oxalique, méthanesulfonique, éthanesulfonique, camphorique et citrique.

Parmi les bases pharmaceutiquement acceptables que l'on peut utiliser pour former un sel d'addition avec les composés de l'invention, on peut citer à titre d'exemples et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

De façon particulière, les radicaux alkyls présents dans la formule (I) peuvent être choisis parmi méthyl, éthyl, n-propyl, isopropyl, n-butyl, isobutyl, sec-butyl, tert-butyl, pentyl, ou hexyl.

Les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy, et hexyloxy.

Les halogènes présents dans la formule (I) peuvent être choisis parmi le brome, le chlore, le fluor, et l'iode.

De façon préférentielle, l'invention concerne les composés suivants:
- 3-méthyl-6-[2-(4-benzylpipéridin-1-yl)éthyl]benzothiazolinone,
- 3-méthyl-6-{2-[N-méthyl N-(2-pipéridin-1-yl-éthyl)aminoéthyl]}benzothiazolinone,
- 3-méthyl-6-[2-(4-phénylpipéridin-1-yl)éthyl]benzothiazolinone,
- 3-méthyl-6-[4-(4-benzylpipéridin-1-yl)-n-but-1-yl]benzothiazolinone,
- 3-méthyl-6-{2-{N-méthyl N-[2-(perhydroazépin-1-yl)-éthyl]amino}éthyl}benzothiazolinone,
- 3-méthyl-6-[4-(4-phénylpipéridin-1-yl)-n-but-1-yl]benzothiazolinone,
- 3-méthyl-6-{4-[N-méthyl N-(2-pipéridin-1-yl-éthyl)amino] n-but-1-yl}benzothiazolinone,
- 3-méthyl-6-{4-[N-méthyl-N-(2-perhydroazépin-1-yl-éthyl)amino-n-but-1-yl}benzothiazolinone,
- 3-méthyl-6-{2-[N-méthyl-N-[2-(1-pyrrolidin-1-yl)éthyl]amino]éthyl}benzothiazolinone,
- 3-méthyl-6-{4-[N-méthyl-N-[2-(1-pyrrolidin-1 -yl)éthyl]amino]n-but-1-yl}benzothiazolinone.

L'invention s'étend également au procédé de préparation des composés de formule (I), caractérisé en ce que on introduit dans une solution appropriée un composé de formule (II) : 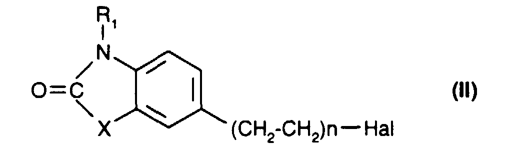 dans laquelle X, R₁ et n sont tels que définis dans la formule (I) et Hal est un atome d'halogène, que l'on condense
- soit avec un composé de formule (III): 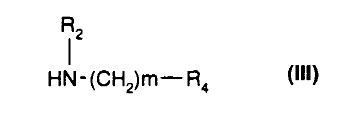 dans laquelle R₂, R₄ et m sont tels que définis dans la formule (I) pour obtenir un composé de formule (Ia) : 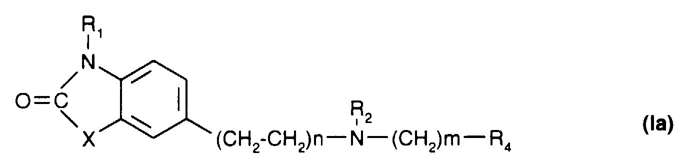 dans laquelle X, R₁, R₂, R₄, n et m sont tels que définis précédemment,
- soit avec un composé de formule (IV) : 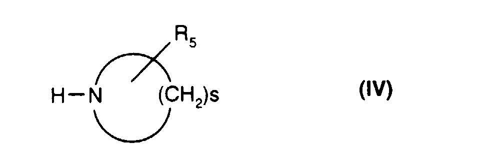 dans laquelle R₅ et s sont tels que définis dans la formule (I),
   pour obtenir un composé de formule (Ib) : 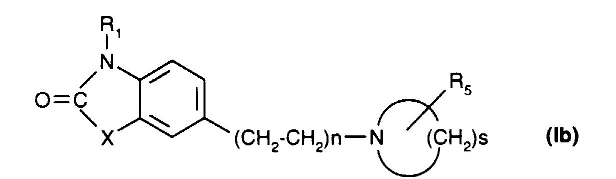 dans laquelle X, R₁, R₅ et s sont tels que définis précédemment,
   composés de formule (I) qui peuvent être, si on le désire,
- purifiés suivant une ou plusieurs méthodes de purification choisies parmi la cristallisation, la chromatographie sur gel de silice, l'extraction, la filtration et le passage sur charbon ou résine,
- séparés, le cas échéant en leurs éventuels isomères optiques,
- ou salifiés par un acide ou une base pharmaceutiquement acceptable.

L'invention concerne également le procédé de préparation des composés de formule (la) tels que définis précédemment caractérisé en ce que un composé de formule (V) : 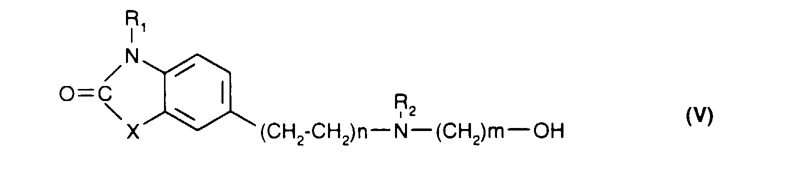 dans laquelle X, R₁, R₂, m et n sont tels que définis dans la formule (I),
est traité successivement par un ou plusieurs agents d'halogénation pour conduire à un composé de formule (VI) : 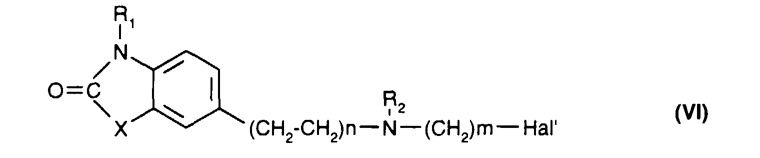 dans laquelle X, R₁, R₂, m et n sont tels que définis précédemment et Hal' représente un atome d'halogène, composé de formule (III) qui est mis en réaction avec un composé de formule (VII) :

H-R₄ **(VII)**

dans laquelle R₄ est tel que défini dans la formule (I),
pour obtenir un composé de formule (la) tel que défini précédemment,
composés de formule (la) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant en leurs éventuels isomères optiques,
- ou salifiés par un acide ou une base pharmaceutiquement acceptable.

L'invention s'étend également au procédé de préparation des composés de formule (la) tels que définis précédemment,
caractérisé en ce que on condense un composé de formule (VIII) : 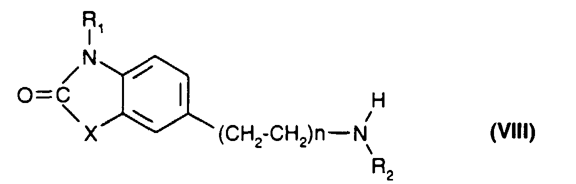 dans laquelle R₁, R₂, X et n sont tels que définis dans la formule (I), avec un composé de formule (IX) :

Hal''-(CH₂)m-R₄ **(IX)**

dans laquelle R₄ et m sont tels que définis dans la formule (I) et Hal" représente un atome d'halogène,
pour obtenir un composé de formule (la) tel que défini précédemment,
composés de formule (la) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant en leurs éventuels isomères optiques,
- ou salifiés par un acide ou une base pharmaceutiquement acceptable.

L'invention concerne également les composés de formule (V) : 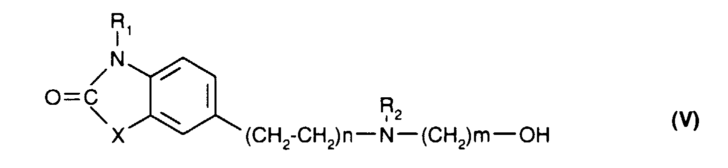 dans laquelle X, R₁, R₂, m et n sont tels que définis dans la formule (I),
et les composés de formule (VI) : 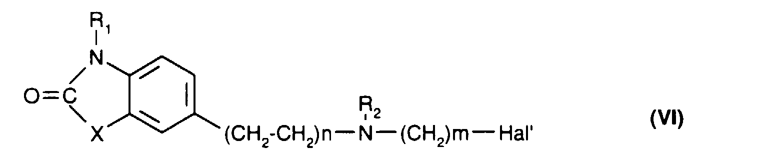 dans laquelle X, R₁, R₂, m et n sont tels que définis dans la formule (I) et Hal' représente un atome d'halogène,
utiles comme intermédiaires de synthèse des composés de formule (I).

Les matières premières utilisées dans les procédés précédemment décrit sont soit commerciales, soit aisément accessibles à l'homme du métier selon des procédés bien connus dans la littérature. On se réfèrera plus particulièrement, pour les composés de formule (II), aux descriptions des demandes de brevets EP 478 446 et EP 506 539.

Les composés de formule (I) possèdent des propriétés pharmacologiques intéressantes qui les rendent utiles pour la prévention et le traitement des pathologies liées aux récepteurs sigma.

La très haute affinité des dérivés de l'invention pour les récepteurs σ les rend utilisables dans le traitement des désordres moteurs, des dystonies (Walker, JM : Drug specificity of pharmacology dystonia, Pharmacol. Biochem. Behav. 1990, 36, 151), de la dyskinésie tardive (Lindstrom, L.H. : Acta Psychiatr. Scand. 1988, 77, 1122), des troubles psychotiques (Chouinard, F., Annable, L. Psychopharmacology 1984, 84, 282), et dans le traitement des dommages liés à l'ischémie périphérique ou cérébrale, des états de choc (Pontecorvo, M.J., Brain Res. Bull. 26, 461), de l'insuffisance circulatoire cérébrale, de la maladie d'Alzheimer, de la régulation des phénomènes immunitaires (Carroll, F.I., Med. Chem. Res. 1992, 2, 3), le traitement des toxicomanies à la cocaïne (Abou - Gharbia, M., Academic. Press. Inc. Bristol. J. Ed. Publisher. 1993, 28, 1), le diagnostic et la localisation des tumeurs (Hudzik, T.J., Psychopharmacology. 1992, 108, 115 ; Abou - Gharbia, M., Academic. Press. Inc. Bristol. J. Ed. Publisher 1993, 28, 1) et le vomissement (Hudzik, T.J., Eur. J. Pharmacol. 1993, 236, 279)) ainsi que dans le traitement des maladies inflammatoires d'origine immunitaire, les troubles de la motricité intestinale, et l'arthrite chronique ou aiguë.

La présente invention a également pour objet les compositions pharmaceutiques contenant les composés de formule (I) ou le cas échéant un de leurs sels d'addition à un acide ou à une base pharmaceutiquement acceptable en combinaison avec un ou plusieurs excipients.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 100 m g par 24 heures en 1 ou 2 prises, plus particulièrement 0,1 à 10 mg,

Les exemples suivants illustrent l'invention.

Les spectres de résonance magnétique nucléaire ¹H ont été réalisés en utilisant le TMS (tétraméthylsilane) comme référence interne. Les déplacements chimiques sont exprimés en partie par million (p.p.m.). Les spectres infrarouge ont été effectués sous forme de pastille de bromure de potassium renfermant environ 1 % du produit à analyser.

### PREPARATION 1 : 3-METHYL 6-(2-PHTALIMIDOETHYL) BENZOTHIAZOLINONE

### Réactifs :

| | |
|---|---|
| 3-méthyl 6-(2-bromoéthyl)benzothiazolinone | 0,01 mole (2,72 g) |
| Phtalimide potassique | 0,01 mole (1,85 g) |
| Diméthylformamide anhydre | 25 cm³ |

### Mode opératoire :

Dans une fiole à col rodé munie d'un réfrigérant à eau et contenant 0,01 mole de phtalimide potassique dans 20 cm³ de diméthylformamide, ajouter goutte à goutte 0,01 mole de 3-méthyl 6-(2-bromoéthyl)benzothiazolinone préalablement solubilisé dans 5 cm³ de diméthylformamide anhydre.

Chauffer à reflux pendant une heure.

Laisser refroidir puis verser le mélange réactionnel dans de l'eau glacée.

Laver plusieurs fois à l'eau le précipité obtenu, l'essorer, le sécher puis le recristalliser.

| | |
|---|---|
| Masse moléculaire | 338,30 g.mole⁻¹ |
| Point de fusion | > 260°C |
| Rendement | 60 % |
| Solvant de recristallisation | Diméthylformamide |

### PREPARATION 2 : 3-METHYL 6-(4-PHTALIMIDOBUTYL)BENZOTHIAZOLINONE

**Réactifs :**

| | |
|---|---|
| 3-méthyl 6-(4-bromobutyl)benzothiazolinone | 0,01 mole (3,00 g) |
| Phtalimide potassique | 0,01 mole (1,85 g) |
| Diméthylformamide | 25 cm³ |

### Mode opératoire :

Il est identique à celui utilisé pour l'obtention du dérivé décrit dans la préparation 1

| | |
|---|---|
| Masse moléculaire | 366,36 g.mole⁻¹ |
| Point de fusion | 159-160°C |
| Rendement | 56 % |
| Solvant de recristallisation | Alcool à 95° |

### PREPARATION 3: CHLORHYDRATE DE 3-METHYL 6-(2-AMINOETHYL)BENZOTHIAZOLINONE

### Réactifs :

| | |
|---|---|
| 3-méthyl 6-(2-phtalimidoéthyl)benzothiazolinone | 0,01 mole (3,38 g) |
| Hydrate d'hydrazine | 0,01 mole (4,85 g) |
| Alcool à 95° | 100 cm³ |

### Mode opératoire :

Chauffer à reflux 0,01 mole de 3-méthyl 6-(2-phtalimidoéthyl)benzothiazolinone dans 100 cm³ d'alcool à 95°

Ajouter goutte à goutte 0,01 mole d'hydrate d'hydrazine. Maintenir le reflux pendant trois heures.

Evaporer l'alcool. Reprendre le résidu par 50 cm³ d'eau et extraire à trois reprises avec 50 cm³ de chloroforme. Sécher les phases chloroformiques sur du chlorure de calcium puis les évaporer sous pression réduite.

Reprendre le résidu par 50 cm³ d'éthanol absolu et faire barboter un courant d'acide chlorhydrique gazeux. Essorer le précipité formé et le recristalliser.

| | |
|---|---|
| Masse moléculaire | 244,75 g.mole⁻¹ pour C₁₀H₁₃ClN₂OS |
| Point de fusion | 228-230°C |
| Rendement | 72 % |
| Solvant de recristallisation | Alcool absolu |

### PREPARATION 4 : CHLORHYDRATE DE 3-METHYL 6-(4-AMINOBUTYL)BENZOTHIAZOLINONE

### Réactifs :

| | |
|---|---|
| 3-méthyl 6-(4-phtalimidobutyl)benzothiazolinone | 0,01 mole (3,66 g) |
| Hydrate d'hydrazine | 0,01 mole (4,85 cm³) |
| Alcool à 95° | 100 cm³ |

### Mode opératoire :

II est identique à celui utilisé pour l'obtention du dérivé décrit dans la préparation 3.

| | |
|---|---|
| Masse moléculaire | 272,80 g.mole⁻¹ |
| Point de fusion | 214-215°C |
| Rendement | 82 % |
| Solvant de recristallisation | Alcool absolu |

### PREPARATION 5 : 3-METHYL 6-(2-TRIFLUOROACETAMIDOETHYL)BENZOTHIAZOLINONE

### Réactifs :

| | |
|---|---|
| Chlorhydrate de 3-méthyl 6-(2-aminoéthyl)benzothiazolinone | 0,010 mole (2,44 g) |
| Anhydride trifluoroacétique | 0,012 mole (1,70 cm³) |
| Pyridine anhydre | 50 cm³ |

### Mode opératoire :

Dans un ballon de 100 cm³, dissoudre 0,010 mole de 3-méthyl 6-(2-aminoéthyl)benzothiazolinone dans 50 cm³ de pyridine anhydre.

Refroidir le mélange réactionnel dans un bain de glace et ajouter goutte à goutte sous agitation magnétique 0,012 mole d'anhydride trifluoroacétique.

Laisser sous agitation pendant trente minutes à température ambiante.

Verser le mélange réactionnel dans l'eau glacée, essorer le précipité formé, le laver à l'eau, le sécher puis le recristalliser.

| | |
|---|---|
| Masse moléculaire | 304,22 g.mole⁻¹ |
| Point de fusion | 156-158°C |
| Rendement | 97 % |
| Solvant de recristallisation | Cyclohexane |

### PREPARATION 6 : 3-METHYL 6-(4-TRIFLUOROACETAMIDOBUTYL)BENZOTHIAZOLINONE

### Réactifs :

| | |
|---|---|
| Chlorhydrate de 3-méthyl 6-(4-aminobutyl)benzothiazolinone | 0,010 mole (2,72 g) |
| Anhydre trifluoroacétique | 0,012 mole (1,70 cm³) |
| Pyridine anhydre | 50 cm³ |

### Mode opératoire :

II est identique à celui utilisé cour l'obtention du dérivé décrit dans la préparation 5.

| | |
|---|---|
| Masse moléculaire | 332,27 g.mole⁻¹ |
| Point de fusion | 114-115°C |
| Rendement | 98 % |
| Solvant de recristallisation | Cyclohexane |

### PREPARATION 7: 3-METHYL 6-[2-(N-METHYLTRIFLUOROACETAMIDO)ETHYL]BENZOTHIAZOLINONE

### Réactifs :

| | |
|---|---|
| 3-méthyl 6-(2-trifluoroacétamidoéthyl)benzothiazolinone | 0,01 mole (3,10 g) |
| Carbonate de potassium | 0,04 mole (5,50 g) |
| Iodure de méthyle | 0,02 mole (1,30 cm³) |
| Diméthylformamide anhydre | 50 cm³ |

### Mode opératoire :

Dans une fiole à col rodé surmontée d'un réfrigérant à eau, introduire 0,01 mole de 3-méthyl 6-(2-trifluoroacétamidoéthyl)benzothiazolinone et 0,04 mole de carbonate de potassium dans 50 cm³ de diméthylformamide anhydre.

Laisser à reflux du solvant sous agitation pendant trente minutes. Ajouter 0,02 mole d'iodure de méthyle.

Après deux heures de réaction à reflux du diméthylformamide, refroidir le mélange réactionnel puis le verser dans 100 cm³ d'eau glacée.

Essorer le précipité formé, le laver à l'eau, le sécher puis le recristalliser.

| | |
|---|---|
| Masse moléculaire | 318,24 g.mole⁻¹ |
| Point de fusion | 122-123°C |
| Rendement | 91 % |
| Solvant de recristallisation | Cyclohexane |

### PREPARATION 8 : 3-METHYL 6-[4-(N-METHYL N-TRIFLUOROACETAMIDO)BUTYL] BENZOTHIAZOLINONE

### Réactifs :

| | |
|---|---|
| 3-méthyl 6-(4-trifluoroacétamidobutyl)benzothiazolinone | 0,01 mole (3,32 g) |
| Carbonate de potassium | 0,04 mole (5,52 g) |
| Iodure de méthyle | 0,02 mole (1,25 cm³) |
| Diméthylformamide anhydre | 50 cm³ |

### Mode opératoire :

Il est identique à celui utilisé pour l'obtention du dérivé décrit dans la préparation 7.

| | |
|---|---|
| Masse moléculaire | 346,30 g.mole⁻¹ |
| Point de fusion | 63-64°C |
| Rendement | 82 % |
| Solvant de recristallisation | Cyclohexane |

### PREPARATION 9 : CHLORHYDRATE DE 3-METHYL 6-[2-(N-METHYLAMINO)ETHYL]BENZOTHIAZOLINONE

### Réactifs :

| | |
|---|---|
| 3-méthyl 6-[2-(N-méthyltrifluoroacétamido)éthyl]benzothiazolinone | 0,01 mole (3,18 g) |
| Carbonate de potassium | 0,04 mole (5,52 g) |
| Mélange méthanol/eau (6 : 1) | 70 cm³ |

### Mode opératoire :

Dans une fiole à col rodé surmontée d'un réfrigérant à eau et contenant 60 cm³ de méthanol et 10 cm³ d'eau, mettre 0,01 mole de 3-méthyl-6-[2-(N-méthyltrifluoroacétamido) éthyl]benzothiazolinone.

Ajouter 0,04 mole de carbonate de potassium. Porter à reflux du mélange de solvants pendant une heure. Evaporer le méthanol, ajouter 50 cm³ d'eau au résidu et extraire à trois reprises par 50 cm³ d'acétate d'éthyle. Sécher les phases organiques réunies sur du carbonate de potassium, filtrer, évaporer le solvant.

Dissoudre le résidu dans 50 cm³ d'acétone anhydre et faire barboter de l'acide chlorhydrique gazeux. Essorer le précipité obtenu, le sécher puis le recristalliser.

| | |
|---|---|
| Masse moléculaire | 258,74 g.mole⁻¹ |
| Point de fusion | 203-204°C |
| Rendement | 80 % |
| Solvant de recristallisation | Acétone anhydre |

### PREPARATION 10 : CHLORHYDRATE DE 3-METHYL 6-[4-(N-METHYLAMINO)BUTYL] BENZOTHIAZOLINONE

**Réactifs :**

| | |
|---|---|
| 3-méthyl 6-[4-(N-méthyltrifluoroacétamido)butyl]benzothiazolinone | 0,01 mole (3,46 g) |
| Carbonate de potassium | 0,04 mole (5,52 g) |
| Mélange méthanol/eau (6:1) | 70 cm³ |

### Mode opératoire :

Il est identique à celui utilisé pour l'obtention du dérivé décrit dans la préparation 9.

| | |
|---|---|
| Masse moléculaire | 286,75 g.mole⁻¹ |
| Point de fusion | 130-132°C |
| Rendement | 80 % |
| Solvant de recristallisation | Acétone anhydre |

### PREPARATION 11 : 3-METHYL-6-{2-[N-(2-HYDROXYETHYL)-N-METHYLAMINO]ETHYL} BENZOTHIAZOLINONE

### Réactifs :

| | |
|---|---|
| 3-méthyl 6-(2-bromoéthyl)benzothiazolinone | 0,032 mole (8,7 g) |
| 2-N-méthyléthanolamine | 0,033 mole (2,6 cm³) |
| Triéthylamine | 0,033 mole (4,6 cm³) |
| Acétone anhydre | 60 cm³ |

### Mode opératoire :

Dans une fiole à col rodé de 250 cm³ contenant 60 cm³ d'acétone anhydre, introduire 0,033 mole de 2-N-méthyléthanolamine, 0,032 mole de 3-méthyl-6-(2-bromoéthyl)benzothiazolinone et 0,033 mole de triéthylamine.

Chauffer le mélange réactionnel à reflux de l'acétone anhydre pendant 15 heures. Laisser refroidir puis évaporer le solvant au bain marie sous pression réduite.

Reprendre le résidu d'évaporation par 30 cm³ d'une solution aqueuse d'acide chlorhydrique 2N. Extraire avec 30 cm³ d'éther éthylique. Alcaliniser la phase aqueuse par une solution aqueuse de soude à 10 %. Extraire de nouveau trois fois avec 50 cm³ de chloroforme. Réunir les fractions chloroformiques, les sécher sur du chlorure de calcium, filtrer puis évaporer au bain marie sous pression réduite.

Reprendre le résidu par 50 cm³ d'éther anhydre et faire barboter de l'acide chlorhydrique gazeux, essorer le précipité formé et le recristalliser.

| | |
|---|---|
| Masse moléculaire | 302,75 g.mole⁻¹ |
| Rendement | 80 % |
| Point de fusion | 55°C |
| Solvant de recristallisation | Acétone |

### PREPARATION 12 : 3-METHYL 6-{4-[N-(2-HYDROXYETHYL)-N-METHYLAMINO]BUTYL} BENZOTHIAZOLINONE

### Réactifs :

| | |
|---|---|
| 3-méthyl 6-(4-bromobutyl)benzothiazolinone | 0,010 mole (3 g) |
| 2-N-méthyléthanolamine | 0,012 mole (1 cm³) |
| Triéthylamine | 0,012 mole (1,66 cm³) |
| Acétone anhydre | 45 cm³ |

### Mode opératoire :

Il est identique à celui utilisé pour l'obtention du dérivé décrit dans la préparation 11.

| | |
|---|---|
| Masse moléculaire | 330,81 g.mole⁻¹ |
| Rendement | 75 % |
| Point de fusion | 60°C |
| Solvant de recristallisation | Acétone |

### PREPARATION 13 : 3-METHYL 6-{2-[N-(2-CHLOROETHYL) N-METHYLAMINO]ETHYL} BENZOTHIAZOLINONE

### Réactifs :

| | |
|---|---|
| Chlorhydrate de 3-méthyl 6-{2-[N-(2-hydroxyethyl)-N-méthylamino]éthyl}benzothiazolinone | 0,011 mole (3,3 g) |
| Chlorure de thionyle | 0,044 mole (3,2 cm³) |
| Chloroforme anhydre | 100 cm³ |

### Mode opératoire :

Dans un ballon à col rodé de 250 cm³ contenant 100 cm³ de chloroforme anhydre, dissoudre à chaud 0,011 mole de chlorhydrate de 3-méthyl 6-{2-[N-(2-hydroxyéthyl) méthylamino]éthyl}benzothiazolinone, ajouter goutte à goutte, 0,044 mole de chlorure de thionyle et chauffer le mélange réactionnel à reflux pendant 1 heure.

Evaporer le mélange chloroforme-chlorure de thionyle au bain marie sous pression réduite.

Reprendre le résidu par 50 cm³ d'éther anhydre, triturer et laisser agiter pendant 30 minutes. Essorer le précipité obtenu et le recristalliser.

| | |
|---|---|
| Masse moléculaire | 321,19 g.mole⁻¹ |
| Rendement | 90 % |
| Point de fusion | 144-147°C |
| Solvant de recristallisation | Acétone |

### PREPARATION 14 : 3-METHYL 6-{4-[N-(2-CHLOROETHYL)N-METHYLAMINO]BUTYL}BENZOTHIAZOLINONE

### Réactifs :

| | |
|---|---|
| Chlorhydrate de 3-méthyl 6-{2-[N-(4-hydroxyethyl)-N-méthylamino]butyl}benzothiazolinone | 0,008 mole (2,6 g) |
| Chlorure de thionyle | 0,033 mole (2,6 cm³) |
| Chloroforme anhydre | 100 cm³ |

### Mode opératoire :

Il est identique à celui utilisé pour l'obtention du dérivé décrit dans la préparation 13.

| | |
|---|---|
| Masse moléculaire | 349,25 g.mole⁻¹ |
| Rendement | 88 % |
| Point de fusion | 176-179°C |
| Solvant de recristallisation | Acétone |

### PREPARATION 15 : 3-METHYL 6-{2-[N-(2-IODOETHYL) N-METHYLAMINO]ETHYL}BENZOTHIAZOLINONE

**Réactifs :**

| | |
|---|---|
| Chlorhydrate de 3-méthyl 6-{2-[N-(2-chloroéthyl)N-méthylamino]éthyl}benzothiazolinone | 0,0068 mole (2,2 g) |
| Iodure de potassium | 0,027 mole (4,5 g) |
| Acétone anhydre | 50 cm³ |

### Mode opératoire :

Dans une fiole à col rodé de 250 cm³ contenant 50 cm³ d'acétone anhydre, introduire 0,0068 mole de chlorhydrate de 3-méthyl 6-{2-[N-(2-chloroéthyl) N-méthylamino]éthyl}benzothiazolinone et 0,027 mole d'iodure de potassium.

Chauffer à reflux de l'acétone pendant 24 heures. Laisser refroidir, filtrer puis évaporer le filtrat au bain marie sous pression réduite.

Reprendre le résidu par de l'éther anhydre, faire barboter de l'acide chlorhydrique gazeux puis essorer le précipité obtenu et le recristalliser.

| | |
|---|---|
| Masse moléculaire | 412,75 g.mole⁻¹ |
| Rendement | 90 % |
| Point de fusion | 124-126°C |

### PREPARATION 16 : 3-METHYL 6-{4-[N-(2-IODOETHYL) N-METHYLAMINO]BUTYL}BENZOTHIAZOLINONE

**Réactifs :**

| | |
|---|---|
| Chlorhydrate de 3-méthyl 6-{4-[N-(2-chloroéthyl)-N-méthylamino]butyl}benzothiazolinone | 0,011 mole (4 g) |
| Carbonate de potassium | 0,044 mole (7,3 g) |
| Acétone anhydre | 60 cm³ |

### Mode opératoire :

Il est identique à celui utilisé pour l'obtention du dérivé décrit dans la préparation 15.

| | |
|---|---|
| Masse moléculaire | 440,79 g.mole⁻¹ |
| Rendement | 88 % |
| Point de fusion | 130-132°C |

### EXEMPLE 1 : 3-METHYL 6-[2-(4-BENZYLPIPERIDIN-1-YL)ETHYL]BENZOTHIAZOLINONE

### Mode opératoire :

Dans une fiole à col rodé de 250 cm³ munie d'un réfrigérant et contenant 70 cm³ d'acétone anhydre, introduire 0,0073 mole (2,00 g) de 3-méthyl 6-(2-bromoéthyl)benzothiazolinone décrite dans la demande EP 478 446, 0,0073 mole (1,30 cm³) de 4-benzylpipéridine et 0,0073 mole (1,00 cm³) de triéthylamine.

Chauffer le mélange à reflux de l'acétone pendant 30 heures. Essorer le précipité de bromhydrate de triéthylamine puis évaporer le filtrat au bain marie sous pression réduite. Reprendre le résidu par une solution aqueuse d'acide chlorhydrique 1N et extraire avec de l'éther.

La phase aqueuse est alcalinisée avec une solution de soude à 10 %. Extraire deux fois à l'éther, réunir les phases éthérées, les sécher sur du chlorure de calcium, filtrer et évaporer sous pression réduite.

Reprendre le résidu par de l'éther anhydre, faire barboter de l'acide chlorhydrique gazeux, essorer le précipité formé et le recristalliser.

| | |
|---|---|
| Masse moléculaire | 402,91 g mol⁻¹ pour C₂₂H₂₇CIN₂OS |
| Rendement | 56 % |
| Point de fusion (chlorhydrate) | 157-159°C |
| Solvant de recristallisation | Acétone |

| **Analyse élémentaire :** | | | |
|---|---|---|---|
| | % C | % H | % N |
| Calculée | 65,58 | 6,75 | 6,95 |
| Trouvée | 65,74 | 6,95 | 6,95 |

| **Spectrométrie dans l'infra-rouge :** | | |
|---|---|---|
| 3400-2500 | cm⁻¹ | ν NH⁺(chlorhydrate) |
| 2960 | cm⁻¹ | ν CH (alkyles) |
| 1680 | cm⁻¹ | ν CO (NCOS) |
| 1590 | cm⁻¹ | ν C=C (aromatiques) |

| **Spectrométrie de résonance magnétique nucléaire (80MHz ; DMSO,d**_{**6**}**) :** | | | |
|---|---|---|---|
| δ = 1,40-1,80 | ppm | (massif, 4H) | (éthyl) |
| δ = 3,20 | ppm | (singulet, 2H) | CH₂-C₆H₅ |
| δ = 7,00-7,50 | ppm | (massif, 8H) | H aromatiques |
| δ = 9,00 | ppm | (signal, 1 H) | NH⁺échangeable dans D₂O |

### EXEMPLE 2 : 3-METHYL 6-[4-(4-BENZYLPIPERIDIN-1-YL)BUTYL]BENZO-THIAZOLINONE

### Réactifs :

| | |
|---|---|
| 3-méthyl 6-(4-bromobutyl)benzothiazolinone | 0,005 mole (1,40 g) |
| 4-benzylpipéridine | 0,005 mole (0,90 cm³) |
| carbonate de potassium | 0,010 mole (1,40 g) |
| Diméthylformamide anhydre | 60 cm³ |

### Mode opératoire :

Il est identique à celui utilisé dans l'exemple 1 en remplaçant la 3-méthyl 6-(2-bromoéthyl)benzothiazolinone par la 3-méthyl 6-(4-bromobutyl)benzothiazolinone décrite dans la demande EP 478446 et la triéthylamine/acétone par le carbonate de potassium/diméthylformamide.

| | |
|---|---|
| Masse moléculaire | 430,98 g.mole⁻¹ pour C₂₄H₃₁CIN₂OS |
| Rendement | 50 % |
| Point de fusion (chlorhydrate) | 160-162°C |
| Solvant de recristallisation | Acétone-éther (2/3) |

| **Analyse élémentaire :** | | | |
|---|---|---|---|
| | %C | %H | % N |
| Calculée | 66,88 | 7,25 | 6,50 |
| Trouvée | 67,02 | 6,98 | 6,30 |

| **Spectrométrie dans l'infra-rouge :** | | |
|---|---|---|
| 3400-2440 | cm⁻¹ | ν NH⁺(chlorhydrate) |
| 3020-2860 | cm⁻¹ | ν CH (alkyles) |
| 1650 | cm⁻¹ | ν CO (NCOS) |
| 1600 | cm⁻¹ | ν C=C (aromatiques) |

| **Spectrométrie de résonance magnétique nucléaire (80MHz ; DMSO,d**_{**6**}**) :** | | | |
|---|---|---|---|
| δ = 6,80-7,40 | ppm | (massif, 8H) | H aromatiques |
| δ = 12,00 | ppm | (signal, 1H) | NH⁺échangeable dans D₂O |

### EXEMPLE 3 : 3-METHYL 6-[2-(4-PHENYLPIPERIDIN-1-YL)ETHYL]BENZOTHIAZOLINONE

### Réactifs :

| | |
|---|---|
| 3-méthyl 6-(2-bromoéthyl)benzothiazolinone | 0,0055 mole (1,5 g) |
| 4-phénylpipéridine | 0,0055 mole (0,88 g) |
| Triéthylamine | 0,0055 mole (0,80 cm³) |
| Acétone anhydre | 40 cm³ |

### Mode opératoire :

II est identique à celui utilisé dans l'exemple 1 en remplaçant la 4-benzyl pipéridine par la 4-phénylpipéridine.

| | |
|---|---|
| Masse moléculaire | 388,80 g.mole⁻¹ pour C₂₁H₂₅CIN₂OS |
| Rendement | 60 % |
| Point de fusion (chlorhydrate) | 260-261 °C |
| Solvant de recristallisation | Alcool absolu |

| **Analyse élémentaire :** pour C₂₁H₂₅CIN₂OS ; 1,25 H₂O, MM = 411,32 g.mole⁻¹ | | | |
|---|---|---|---|
| | %C | %H | %N |
| Calculée | 61,31 | 6,12 | 6,81 |
| Trouvée | 61,16 | 6,16 | 6,73 |

| **Spectrométrie dans l'infra-rouge :** | | |
|---|---|---|
| 3000-2880 | cm⁻¹ | ν CH(alkyles) |
| 2500 | cm⁻¹ | ν NH⁺ (chlorhydrate) |
| 1680 | cm⁻¹ | ν CO (NCOS) |
| 1600-1570 | cm⁻¹ | ν C=C (aromatiques) |

| **Spectrométrie de résonance magnétique nucléaire (80MHz ; DMSO,d**_{**6**}**) :** | | | |
|---|---|---|---|
| δ = 1,60-2,20 | ppm | (massif, 4H) | (éthyl) |
| δ = 3,00-3,80 | ppm | (massif, 12H) | NCH₃ et pipéridine |
| δ = 7,10-7,70 | ppm | (massif, 8H) | H aromatiques |
| δ = 10,50 | ppm | (signal, 1 H) | NH⁺échangeable dans D₂O |

### EXEMPLE 4 : 3-METHYL 6-[4-(4-PHENYLPIPERIDIN-1-YL)BUTYL] BENZOTHIAZOLINONE

### Réactifs :

| | |
|---|---|
| 3-méthyl 6-(4-bromobutyl)benzothiazolinone | 0,0073 mole (2,00 g) |
| 4-phénylpipéridine | 0,0073 mole (1,20 cm³) |
| Triéthylamine | 0,0073 mole (1,00 cm³) |
| Acétone anhydre | 70 cm³ |

### Mode opératoire :

Il est identique à celui utilisé dans l'exemple 2 en remplaçant la 4-benzyl-pipéridine par la 4-phényl pipéridine.

| | |
|---|---|
| Masse moléculaire | 416,93 g.mole⁻¹ pour C₂₃H₂₉CIN₂OS |
| Rendement | 43 % |
| Point de fusion (chlorhydrate) | 210-212°C |
| Solvant de recristallisation | Alcool absolu |

| **Analyse élémentaire** : pour C₂₃H₂₉CIN₂OS ; 0,5 H₂O, MM = 425,94 g.mole⁻¹ | | | |
|---|---|---|---|
| | %C | %H | % N |
| Calculée | 64,85 | 6,86 | 6,57 |
| Trouvée | 64,99 | 7,10 | 6,64 |

| **Spectrométrie dans l'infra-rouge :** | | |
|---|---|---|
| 3020-2840 | cm⁻¹ | ν CH(alkyles) |
| 2500 | cm⁻¹ | ν NH⁺ (chlorhydrate) |
| 1670 | cm⁻¹ | ν CO (NCOS) |
| 1590 | cm⁻¹ | ν C=C (aromatiques) |

| **Spectrométrie de résonance magnétique nucléaire (300MHz ; DMSO,d**_{**6**}**) :** | | | |
|---|---|---|---|
| δ = 1,50-1,80 | ppm | (massif, 4H) | CH₂-CH₂-CH₂-CH₂ |
| δ = 3,40 | ppm | (singulet, 3H) | NCH₃ |
| δ = 7,20-7,70 | ppm | (massif, 8H) | H aromatiques |
| δ = 10,50 | ppm | (signal, 1 H) | NH⁺ échangeable dans D₂O |

### EXEMPLES 5 A 8 :

En procédant de la même façon que pour les exemples 1 à 4, mais en remplaçant la 3-méthyl 6-(2-bromoéthyl)benzothiazolinone et la 3-méthyl 6-(4-bromobutyl)benzothiazolinone par la 3-méthyl 6-(2-bromoéthyl)benzoxazolinone et la 3-méthyl 6-(4-bromobutyl)benzoxazolinone décrites dans le brevet EP 506 539, on obtient respectivement les composés des exemples suivants:

### EXEMPLE 5 : 3-METHYL 6-[2-(4-BENZYLPIPERIDIN-1-YL)ETHYL]BENZOXAZOLINONE

### EXEMPLE 6 : 3-METHYL 6-[4-(4-BENZYLPIPERIDIN-1-YL)BUTYL]BENZOXAZOLINONE

### EXEMPLE 7: 3-METHYL 6-[2-(4-PHENYLPIPERIDIN-1-YL)ETHYL]BENZOXAZOLINONE

### EXEMPLE 8 : 3-METHYL 6-[4-(4-PHENYLPIPERIDIN-1-YL)BUTYL]BENZOXAZOLINONE

### EXEMPLE 9 : 3-METHYL 6-{2-[4-(4-FLUOROBENZYL)PIPERIDIN-1-YL]ETHYL}BENZOTHIAZOLINONE

En procédant comme dans l'exemple 1, mais en remplaçant la 4-benzylpipéridine par la 4-(4-fluorobenzyl)pipéridine, on obtient le composé du titre.

### EXEMPLE 10 : 3-METHYL 6-{2-[4-(4-FLUOROBENZYL)PIPERIDIN-1-YL]ETHYL}BENZOXAZOLINONE

En procédant de la même façon que pour l'exemple 9, mais en remplaçant la 3-méthyl 6-(2-bromoéthyl)benzothiazolinone par la 3-méthyl 6-(2-bromoéthyl) benzoxazolinone, on obtient le composé du titre.

### EXEMPLE 11 : 3-METHYL 6-{2-[N-(2-PYRROLIDIN-1-YL-ETHYL) N-METHYLAMINO] ETHYL}BENZOTHIAZOLINONE

### Réactifs :

| | |
|---|---|
| Chlorhydrate de la 3-méthyl 6-{2-[N-(2-iodoéthyl) N-méthylamino]éthyl}benzothiazolinone (préparation 15) | 0,0041 mole (1,73 g) |
| Pyrrolidine | 0,0041 mole (0,40 cm³) |
| Triéthylamine | 0,0082 mole (1,20 cm³) |
| Acétone anhydre | 50 cm³ |

### Mode opératoire :

II est identique à celui utilisé dans l'exemple 1 en remplaçant d'une part la 4-benzylpipéridine par la pyrrolidine et d'autre part la 3-méthyl 6-(2-bromoéthyl)benzothiazolinone par le chlorhydrate de la 3-méthyl 6-{2-[N-(2-iodoéthyl) N-méthylamino]éthyl}benzothiazolinone.

| | |
|---|---|
| Masse moléculaire | 392,32 g.mole⁻¹ pour C₁₇H₂₇Cl₂N₃OS |
| Rendement | 50 % |
| Point de fusion (dichlorhydrate) | 250-252°C |
| Solvant de recristallisation | Alcool absolu |

| **Analyse élémentaire :** | | | |
|---|---|---|---|
| | % C | %H | %N |
| Calculée | 52,04 | 6,94 | 10,71 |
| Trouvée | 51,78 | 7,00 | 10,61 |

| **Spectrométrie dans l'infra-rouge :** | | |
|---|---|---|
| 3400-2500 | cm⁻¹ | ν NH⁺(chlorhydrate) |
| 3000-2800 | cm⁻¹ | ν CH (alkyles) |
| 1670 | cm⁻¹ | ν CO (NCOS) |
| 1600 | cm⁻¹ | ν C=C (aromatiques) |

| **Spectrométrie de résonance magnétique nucléaire (300MHz ; DMSO,d**_{**6**}**) :** | | | |
|---|---|---|---|
| δ = 2,90 | ppm | (singulet, 3H) | NCH₃ (amine) |
| δ = 3,40 | ppm | (singulet, 3H) | NCH₃ (thiocarbamate) |
| δ = 7,30-7,40 | ppm | (massif, 2H) | H_{4,5} (benzothiazolinone) |
| δ = 7,60 | ppm | (singulet, 1H) | H₇ (benzothiazolinone) |
| δ = 10,10 | ppm | (signal, 2H) | 2NH₂+échangeables dans D₂O |

### EXEMPLE 12 : 3-METHYL 6-{2-[N-(2-PIPERIDIN-1-YL-ETHYL)N-METHYLAMINO]ETHYL} BENZOTHIAZOLINONE

### Mode opératoire :

Dans une fiole à col rodé de 250 cm³ contenant 50 cm³ d'acétone anhydre, introduire 0,0041 mole (1,08 g) de 3-méthyl 6-(2-(N-méthylamino)éthyl)benzothiazolinone (préparation 9), 0,0041 mole (0,75 g) de chlorhydrate de 1-(2-chloroéthyl)pipéridine et 0,0082 mole (1,20 cm³) de triéthylamine.

Chauffer à reflux le mélange réactionnel pendant 48 heures. Filtrer et évaporer l'acétone au bain marie sous pression réduite. Reprendre le résidu par 50 cm³ d'une solution aqueuse d'acide acétique, extraire trois fois avec 40 cm³ de chloroforme. Alcaliniser la phase aqueuse avec une solution de bicarbonate de potassium à 10 %. Extraire trois fois avec 50 cm³ de chloroforme. Sécher les phases chloroformiques réunies sur du chlorure de calcium, filtrer puis évaporer au bain marie sous pression réduite.

Reprendre le résidu par 30 cm³ d'éther anhydre et faire barboter de l'acide chlorhydrique gazeux, essorer le précipité formé et le recristalliser.

| | |
|---|---|
| Masse moléculaire | 406,35 g.mole⁻¹ pour C₁₈H₂₉Cl₂N₃OS |
| Rendement | 50 % |
| Point de fusion (dichlorhydrate) | > 260°C |
| Solvant de recristallisation | Alcool absolu |

| **Analyse élémentaire :** | | | |
|---|---|---|---|
| | % C | % H | % N |
| Calculée | 53,20 | 7,19 | 10,34 |
| Trouvée | 53,02 | 7,02 | 10,52 |

| **Spectrométrie dans l'infra-rouge :** | | |
|---|---|---|
| 3400-2500 | cm⁻¹ | ν NH⁺(chlorhydrate) |
| 3000-2800 | cm⁻¹ | ν CH (alkyles) |
| 1670 | cm⁻¹ | ν CO (NCOS) |
| 1600 | cm⁻¹ | ν C=C (aromatiques) |

| **Spectrométrie de résonance magnétique nucléaire (80MHz ; DMSO,d**_{**6**}**) :** | | | |
|---|---|---|---|
| δ = 2,80 | ppm | (singulet, 3H) | NCH₃ (amine) |
| δ = 7,30-7,40 | ppm | (massif, 2H) | H_{4,5} (benzothiazolinone) |
| δ = 7,60 | ppm | (multiplet, 1 H) | H₇ (benzothiazolinone) |
| δ = 10,8-11,10 | ppm | (signal, 2H) | 2NH⁺échangeables dans D₂O |

### EXEMPLE 13 : 3-METHYL 6-{2-[N-(2-PERHYDROAZEPIN-1-YL-ETHYL)N-METHYLAMINO]ETHYL}BENZOTHIAZOLINONE

### Réactifs :

| | |
|---|---|
| 3-méthyl 6-[2-(N-méthylamino)éthyl]benzothiazolinone | 0,0042 mole (1,1 g) |
| Chlorhydrate de 1-(2-chloroéthyl)perhydroazépine | 0,0042 mole (0,9 g) |
| Triéthylamine | 0,0042 mole (0,6 cm³) |
| Acétone anhydre | 50 cm³ |

### Mode opératoire :

Il est identique à celui utilisé dans l'exemple 12 en remplaçant la 1-(2-chloroéthyl)pipéridine par la 1-(2-chloroéthyl)-perhydroazépine.

| | |
|---|---|
| Masse moléculaire | 420,37 g.mole⁻¹ pour C₁₉H₃₁Cl₂N₃OS |
| Rendement | 30 % |
| Point de fusion (dichlorhydrate) | 262-264°C |
| Solvant de recristallisation | Acétone-éther (2/3) |

| **Analyse élémentaire :** | | | |
|---|---|---|---|
| | %C | %H | %N |
| Calculée | 54,28 | 7,32 | 9,99 |
| Trouvée | 54,38 | 7,16 | 9,70 |

| **Spectrométrie dans l'infra-rouge :** | | |
|---|---|---|
| 2920 | cm⁻¹ | ν CH (alkyles) |
| 2600-2300 | cm⁻¹ | ν NH+ (chlorhydrate) |
| 1670 | cm⁻¹ | ν CO (NCOS) |
| 1600 | cm⁻¹ | ν C=C (aromatiques) |

### EXEMPLE 14: 3-METHYL 6-{4-[N-(2-PYRROLIDIN-1-YL-ETHYL)N-METHYLAMINO] BUTYL}BENZOTHIAZOLINONE

### Réactifs :

| | |
|---|---|
| Chlorhydrate de 3-méthyl-6-{4-[N-(2-iodoéthyl) N-méthylamino]butyl}benzothiazolinone | 0,0074 mole (1,50 g) |
| Pyrrolidine | 0,0074 mole (0,62 cm³) |
| Triéthylamine | 0,0150 mole (2,00 cm³) |
| Acétone anhydre | 50 cm³ |

### Mode opératoire :

II est identique à celui utilisé dans l'exemple 1 mais en remplaçant la 4-benzyl pipéridine par la pyrrolidine et la 3-méthyl 6-(2-bromoéthyl)benzothiazolinone par le chlorhydrate de 3-méthyl-6-{4-[N-(2-iodoéthyl) N-méthylamino]butyl}benzothiazolinone (préparation 16).

| | |
|---|---|
| Masse moléculaire | 420,37 g.mole⁻¹ pour C₁₉H₃₁Cl₂N₃OS |
| Rendement | 55 % |
| Point de fusion (dichlorhydrate) | 244-246°C |
| Solvant de recristallisation | Alcool absolu |

| **Analyse élémentaire :** | | | |
|---|---|---|---|
| | % C | %H | % N |
| Calculée | 54,23 | 7,13 | 9,99 |
| Trouvée | 54,13 | 7,20 | 10,14 |

| **Spectrométrie dans l'infra-rouge :** | | |
|---|---|---|
| 3400-2400 | cm⁻¹ | ν NH⁺ (chlorhydrate) |
| 2900 | cm⁻¹ | ν CH (alkyles) |
| 1670 | cm⁻¹ | ν CO (NCOS) |
| 1600 | cm⁻¹ | ν C=C (aromatiques) |

| **Spectrométrie de résonance magnétique nucléaire (300MHz ; DMSO,d**_{**6**}**) :** | | | |
|---|---|---|---|
| δ = 2,70 | ppm | (multiplet, 2H) | (benzothiazolinone-CH₂) |
| δ = 2,90 | ppm | (singulet, 3H) | NCH₃(amine) |
| δ = 3,00-3,30 | ppm | (massif, 4H) | CH₂-N(CH₃)-CH₂ |
| δ = 7,20-7,30 | ppm | (massif, 2H) | H_{4,5} (benzothiazolinone) |
| δ = 7,60 | ppm | (singulet, 1H) | H₇ (benzothiazolinone) |
| δ = 10,90 | ppm | (signal, 1H) | NH⁺échangeable dans D₂O |
| δ = 11,20 | ppm | (signal, 1H) | NH⁺échangeable dans D₂O |

### EXEMPLE 15 : 3-METHYL 6-{4-[N-(2-PIPERIDIN-1-YL-ETHYL)N-METHYLAMINO] BUTYL}BENZOTHIAZOLINONE

### Réactifs :

| | |
|---|---|
| 3-méthyl-6-[4-(N-méthylamino)butyl]benzothiazolinone | |
| (préparation 10) | 0,0037 mole (1,5 g) |
| Chlorhydrate de 1-(2-chloroéthyl)pipéridine | 0,0037 mole (0,36 cm³) |
| Triéthylamine | 0,0037 mole (0,52 cm³) |
| Acétone anhydre | 50 cm³ |

### Mode opératoire :

II est identique à celui utilisé dans l'exemple 1 en remplaçant la 4-benzylpipéridine par le chlorhydrate de 1-(2-chloroéthyl)pipéridine et la 3-méthyl 6-(2-bromoéthyl)benzothiazolinone par la 3-méthyl 6-[4-(N-méthylamino)butyl]benzothiazolinone (préparation 10).

| | |
|---|---|
| Masse moléculaire | 422,37 g.mole⁻¹ pour C₂₀H₃₃Cl₂N₃OS |
| Rendement | 55 % |
| Point de fusion (dichlorhydrate) | 252-254°C |
| Solvant de recristallisation | Alcool absolu |

| **Analyse élémentaire :** | | | |
|---|---|---|---|
| | %C | %H | % N |
| Calculée | 54,02 | 7,87 | 9,94 |
| Trouvée | 54,37 | 7,71 | 9,56 |

| **Spectrométrie dans l'infra-rouge :** | | |
|---|---|---|
| 3440-2480 | cm⁻¹ | ν NH⁺ (chlorhydrate) |
| 2990 | cm⁻¹ | ν CH (alkyles) |
| 1670 | cm⁻¹ | ν CO (NCOS) |
| 1600 | cm⁻¹ | ν C=C (aromatiques) |

| **Spectrométrie de résonance magnétique nucléaire (300MHz ; DMSO,d**_{**6**}**) :** | | | |
|---|---|---|---|
| δ = 2,60-2,90 | ppm | (massif, 7H) | CH₂-N(CH₃)-CH₂ |
| δ = 7,20-7,30 | ppm | (massif, 2H) | H_{4,5} (benzothiazolinone) |
| δ = 7,50 | ppm | (singulet, 1H) | H₇ (benzothiazolinone) |
| δ = 10,70 | ppm | (signal, 2H) | 2NH⁺échangeables dans D₂O |

### EXEMPLE 16 : 3-METHYL 6-{4-[N-(2-PERHYDROAZEPIN-1-YL-ETHYL)-N-METHYLAMINO]BUTYL}BENZOTHIAZOLINONE

### Réactifs :

| | |
|---|---|
| 3-méthyl 6-[4-(N-méthylamino)butyl]benzothiazolinone | 0,0042 mole (1,10 g) |
| Chlorhydrate de 1-(2-chloroéthyl)-perhydroazépine | 0,0084 mole (1,80 g) |
| Triéthylamine | 0,0084 mole (1,20 cm³) |
| Acétone anhydre | 50 cm³ |

**Mode opératoire :** il est identique à celui utilisé dans l'exemple 12 mais en remplaçant la 3-méthyl 6-[2-(N-méthylamino)éthyl]benzothiazolinone par la 3-méthyl 6-[4-(N-méthylamino) butyl]benzothiazolinone et la 1-(2-chloroéthyl)pipéridine par la 1-(2-chloroéthyl) perhydroazépine.

| | |
|---|---|
| Masse moléculaire | 448,43 g.mole⁻¹ pour C₂₁H₃₅Cl₂N₃OS |
| Rendement | 45 % |
| Point de fusion (dichlorhydrate) | 232-234°C |
| Solvant de recristallisation | Acétone-éther (2/3) |

| **Analyse élémentaire :** pour C₂₁H₃₅Cl₂N₃OS ; 0,75 H₂O, MM = 461,94 g.mole⁻¹ | | | |
|---|---|---|---|
| | %C | %H | %N |
| Calculée | 54,59 | 7,80 | 9,09 |
| Trouvée | 54,71 | 7,42 | 9,05 |

| **Spectrométrie dans l'infra-rouge :** | | |
|---|---|---|
| 2600-2300 | cm⁻¹ | ν NH⁺ (chlorhydrate) |
| 2920 | cm⁻¹ | ν CH (alkyles) |
| 1670 | cm⁻¹ | ν CO (NCOS) |
| 1600 | cm⁻¹ | ν C=C (aromatiques) |

| **Spectrométrie de résonance magnétique nucléaire (80MHz ; DMSO,d**_{**6**}**) :** | | | |
|---|---|---|---|
| δ = 3,50-3,60 | ppm | (massif, 4H) | CH₂-N(CH₃)-CH₂ |
| δ = 7,20-7,30 | ppm | (massif, 2H) | H_{4,5} (benzothiazolinone) |
| δ = 7,60 | ppm | (singulet, 1H) | H₇ (benzothiazolinone) |
| δ = 10,90 | ppm | (signal, 2H) | 2NH⁺échangeables dans D₂O |

### EXEMPLE 17 : 3-METHYL 6-{4-[N-(CYCLOPROPYLAMINOETHYL) N-METHYL AMINO]BUTYL}BENZOTHIAZOLINONE

En procédant comme dans l'exemple 14 mais en remplaçant la pyrrolidine par la cyclopropylamine, on obtient le produit du titre.

### EXEMPLE 18 : 3-METHYL 6-{4-[N-(CYCLOHEXYLAMINOETHYL) N-METHYL AMINO]BUTYL}BENZOTHIAZOLINONE

En procédant comme dans l'exemple 14 mais en remplaçant la pyrrolidine par la cyclohexylamine, on obtient le produit du titre.

### EXEMPLE 19 : 3-METHYL 6-{4-[N-(n,n-DIPROPYLAMINOETHYL) N-METHYL AMINO]BUTYL}BENZOTHIAZOLINONE

En procédant comme dans l'exemple 14 mais en remplaçant la pyrrolidine par la di-n-propylamine, on obtient le produit du titre.

### EXEMPLES 20 A 30 :

En procédant comme dans les exemples 1 à 19 mais en utilisant les dérivés non-méthylés en 3 de la benzothiazolinone ou de la benzoxazolinone, on obtient les composés des exemples suivants:

### EXEMPLE 20 : 6-[2-(4-BENZYLPIPERIDIN-1-YL) ETHYL]BENZOTHIAZOLINONE

### EXEMPLE 21 : 6-[4-(4-BENZYLPIPERIDIN-1-YL) BUTYL]BENZOTHIAZOLINONE

### EXEMPLE 22 : 6-[2-(4-PHENYLPIPERIDIN-1-YL) ETHYL]BENZOTHIAZOLINONE

### EXEMPLE 23 : 6-[4-(4-PHENYLPIPERIDIN-1-YL)-1-YL)BUTYL]BENZOTHIAZOLINONE

### EXEMPLE 24 : 6-[2-(4-BENZYLPIPERIDIN-1-YL) ETHYL]BENZOXAZOLINONE

### EXEMPLE 25 : 6-[4-(4-BENZYLPIPERIDIN-1-YL) BUTYL]BENZOXAZOLINONE

### EXEMPLE 26 : 6-[2-(4-PHENYLPIPERIDIN-1-YL) ETHYL]BENZOXAZOLINONE

### EXEMPLE 27 : 6-[4-(4-PHENYLPIPERIDIN-1-YL) BUTYL]BENZOXAZOLINONE

### EXEMPLE 28 : 6-{2-[4-(4-FLUOROBENZYL)PIPERIDIN-1-YL]ETHYL]BENZOTHIAZOLINONE

### EXEMPLE 29 : 6-{2-[4-(4-FLUOROBENZYL)PIPERIDIN-1-YL]ETHYL}BENZOXAZOLINONE

### EXEMPLE 30 : 6-{2-[N-(2-PYRROLIDIN-1-YL-ETHYL) N-METHYLAMINO]ETHYL}BENZOTHIAZOLINONE

### EXEMPLE 31 : 6-{2-[N-(2-PIPERIDIN-1-YL-ETHYL) N-METHYLAMINO]ETHYL}BENZOTHIAZOLINONE

### EXEMPLE 32 : 6-{2-[N-(2-PERHYDROAZEPIN-1-YL-ETHYL) N-METHYLAMINO] ETHYL}BENZOTHIAZOLINONE

### EXEMPLE 33 : 6-{4-[N-(2-PYRROLIDIN-1-YL-ETHYL) N-METHYLAMINO]BUTYL} BENZOTHIAZOLINONE

### EXEMPLE 34 : 6-{4-[N-(2-PIPERIDIN-1-YL-ETHYL) N-METHYLAMINO]BUTYL} BENZOTHIAZOLINONE

### EXEMPLE 35 : 6-{4-[N-(2-PERHYDROAZEPIN-1-YL)ETHYL] N-METHYLAMINO] BUTYL}BENZOTHIAZOLINONE

### EXEMPLE 36 : 6-{4-[N-(CYCLOPROPYLAMINOETHYL) N-METHYLAMINO]BUTYL} BENZOTHIAZOLINONE

### EXEMPLE 37 : 6-{4-[N-(CYCLOHEXYLAMINOETHYL) N-METHYLAMINO]BUTYL} BENZOTHIAZOLINONE

### EXEMPLE 38 : 6-{4-[N-(N,N-DI n-PROPYLAMINOETHYL) N-METHYLAMINO] BUTYL}BENZOTHIAZOLINONE

### ETUDE PHARMACOLOGIQUE DES DERIVES DE L'INVENTION

### EXEMPLE A : ETUDE DE LA TOXICITE AIGUE

La toxicité aiguë a été appréciée après administration orale à des lots de 8 souris (26 ± 2 grammes) d'une dose de 100 mg.kg⁻¹ des composés de l'invention. Les animaux ont été observés à intervalles réguliers au cours de la première journée et quotidiennement pendant les deux semaines suivant le traitement.
Il apparaît que les composés de l'invention sont totalement atoxiques. Ils n'entrainent aucun décès après administration à une dose de 100 mg.kg⁻¹ et on ne constate pas de troubles après administration de cette dose.

### EXEMPLE B : BILAN RECEPTORIEL D'AFFINITE IN VITRO

Les produits sont testés sur chaque récepteur à 5 concentrations différentes (10⁻⁵M, 10⁻⁶M, 10⁻⁷M, 10⁻⁸M, 10⁻⁹M) en triplicata. Lorsque le coefficient de liaison IC₅₀ est inférieur à une concentration de 10⁻⁶M, le Ki est mesuré en utilisant 12 concentrations du produit.

Le tableau ci-dessous regroupe les récepteurs pour lesquels l'affinité des composés de l'invention à été déterminée, le tissu choisi, la concentration retenue pour déterminer la liaison non spécifique et le radioligand utilisé pour marquer le récepteur.

| **Récepteur ou site** | **Radioligand** | **Liaison non spécifique** | **Tissu** |
|---|---|---|---|
| 5-HT_{1A} | 8-OH DPAT | 10⁻⁵M Buspirone | Hippocampe de boeuf + cortex frontal |
| 5-HT_{1B} | [³H] Cyanopindolol ou 5 OH Tryptamine | 10⁻⁵M Sérotonine froide ou propanolol | Cerveau de rat (cortex frontal + striatum) |
| 5-HT_{1C} | N-méthyl mésulergine | 10⁻⁵M Miansérine | Plexus chroroïde porc |
| 5-HT₂ | [³H] kétansérine | 10⁻⁵M Spipérone | Cortex frontal boeuf |
| 5-HT3 | [³H] Quipazine ou BRL 43694 | 10⁻⁵M ICS 255930 | cellules NG 108-15 |
| α₁ | [³H] Prazosin | 10⁻⁵M Phentolamine | Cerveau de rat |
| α₂ | [³H] Rauwolfine | 10⁻⁵M Yohimbine | Cerveau de rat |
| D₁ | [³H] SCH 23390 | 10⁻⁶M Butaclamol | Striatum de boeuf |
| D₂ | [³H] Raclopride | 10⁻⁶M Halopéridol (10⁻5 M spipérone) | Striatum de boeuf |
| M1 | [³H] Télenzépine | 10⁻⁵M Atropine | Cortex de rat |
| H₁ | [³H] Pyrilamine | 10⁻⁶M Chlorphéniramine | Cortex de veau |
| σ (sigma) | [³H] DTG | 10⁻⁶M 3-PPP | Hippocampe de veau |

Les résultats du test ont montré que les composés de l'invention sont de puissants et sélectifs ligands des récepteurs σ (sigma). A titre de comparaison les dérivés de la présente invention ont une affinité 100 fois plus puissante que les dérivés de la demande EP 478 446 pour le récepteur sigma.

Par ailleurs, les dérivés de la présente invention sont également beaucoup plus sélectifs que les dérivés de la demande EP 478 446. En particulier, ils sont 100 à 1 000 fois moins affins pour les récepteurs D₂ que les dérivés de la demande EP 478 446.

### EXEMPLE C : ANTAGONISME DE l'HYPERMOTILITE INDUITE PAR L'AMPHETAMINE

On injecte à des groupes de 10 souris NMRI-CERJ par voie intrapéritonéale (IP) 4 mg kg⁻¹ de d-amphétamine juste après l'injection IP du composé à tester, et on place les souris dans un actimètre pendant 30 minutes.
Le nombre d'interruptions des cellules photélectriques est compté, de même que le comportement stéréotypé.
L'activité des composés testés est exprimée en pourcentage de l'antagonisme de l'hyperactivité induite par l'amphétamine.
L'exemple suivant montre que les composés de l'invention sont de puissants antagonistes de l'hypermotilité induite par l'amphétamine ce qui permet de conclure à une activité dans les troubles du système nerveux central pour les produits de l'invention.

| **PRODUIT** | **Dose (mg.kg**^{**-1**}**) IP** | | |
|---|---|---|---|
| | **2** | **4** | **8** |
| 3-méthyl 6-[2-(4-benzylpipéridin-1-yl)éthyl]benzothiazolinone | 23 % | 50 % | 63 % |
| ***Exemple 1*** | | | |

### EXEMPLE D : RECHERCHE D'EFFET CATALEPSIGENE

L'effet catalepsigène est recherché chez le rat par voie intrapéritonéale. Ce test est prédictif de l'existence d'effets secondaires du type extrapyramidaux. 6 groupes de rats Wistar ont reçu une injection des composés de l'invention et sont ensuite testés pour leur activité catalepsigène à 30 minutes d'intervalle. L'halopéridol est utilisé comme référence.

Les résultats suivants sont exprimés en pourcentage du score maximal possible

Les résultats montrent que les composés de l'invention n'ont pas de pouvoir catalepsigène comparé à l'halopéridol qui produit à la dose de 2 mg.kg⁻¹ un effet catalepsigène de 95 % dans les mêmes conditions d'études. Ce résultat confirme l'absence d'effets secondaires de type extrapyramidaux des produits de l'invention, ce qui pouvait être déduit des résultats de liaisons réceptorielles (exemple B).

### EXEMPLE E : ETUDE DE L'ACTIVITE ANTIDEPRESSIVE DES COMPOSES DE L'INVENTION

### PRINCIPE :

L'étude des produits est réalisée sur le modèle du "renoncement appris" qui consiste à induire chez l'animal, par une série d'évènements aversifs incontrôlables, un déficit lors des tâches d'évitement ultérieures.

### PROTOCOLE :

Ce test a été mis au point par Sherman A.D., Sacquitne J.L., et Petty F. (Pharmacol. Biochem. Behav., 1982, 16, 449-454). Nous utilisons des rats mâles Wistar d'un poids compris entre 180 et 200 grammes. Les animaux sont maintenus à l'animalerie une semaine avant le test, dans des boîtes en plastique, par groupes de 10, à une température ambiante de 21°C ± 1°C, avec libre accès à l'eau et à la nourriture.
Les animaux sont ensuite isolés dans des boîtes de petites dimensions et sont soumis à 60 chocs électriques inévitables (0,8 mA toutes les minutes ± 15 secondes). Un groupe de rats témoins ne reçoit pas de chocs électriques.
La capacité des animaux à réaliser un apprentissage d'évitement (passage d'un compartiment à l'autre, afin d'éviter les chocs électriques) est appréciée 48 heures après et pendant 3 jours consécutifs. Lors des séances d'apprentissage, les animaux subissent 2 essais par minute pendant 15 minutes. Le nombre d'échecs d'échappement est noté pour chaque rat. Les animaux sont traités (i.p. : 0,5 cm³/100 g) à jeun 6 heures après les chocs inévitables et 4 jours de suite, le matin 30 minutes avant la séance d'apprentissage et le soir entre 18 et 19 h.
Les produits étudiés sont mis en solution dans de l'eau distillée.
Les produits étudiés sont administrés aux doses 0,05 mg.kg⁻¹/jour.

### RESULTATS :

Le test prouve que certains produits de l'invention diminuent significativement le nombre d'échecs d'échappement ce qui traduit, pour certains produits de l'invention, une forte activité de type antidépressive.

### EXEMPLE F : ETUDE DE L'ACTIVITE ANXIOLYTIQUE - TEST DIT DES CAGES CLAIRE/OBSCURE CHEZ LA SOURIS

### PRINCIPE :

Nous nous proposons d'étudier les effets anxiolytiques des composés de l'invention, au moyen du test des cages claires/obscures chez la souris.

### PROTOCOLE :

Ce test a été mis au point par Crawley et al. (Pharmacol. Biochem. Behav. 1981, 15 (5), pp 695-9), puis modifié et comportementalement validé.

Il s'agit de deux cages de taille égale (20 X 20 X 14 cm) en PVC. L'une est fortement éclairée par une lampe de 100 W(lumière "froide"), l'autre est obscurcie. Les deux cages sont séparées l'une de l'autre au moyen d'un petit tunnel opaque (5 X 7 cm). Les souris sont individuellement introduites dans la cage obscure, on enregistre, au moyen de claviers reliés à un ordinateur, durant 5 minutes, le temps passé par les animaux dans la cage éclairée ainsi que le nombre des transitions entre la cage obscure et la cage éclairée.

Chaque groupe expérimental comprend au minimum 15 animaux.

### RESULTATS :

L'administration, par voie intrapéritonéale de certains produits de l'invention entraine une augmentation du temps passé par les souris dans la cage éclairée et du nombre des transitions entre la cage obscure et la cage éclairée.

Cette augmentation significative des deux paramètres étudiés montrent la remarquable activité anxiolytique de certains composés de l'invention.

### EXEMPLE G : RECHERCHE D'UNE ACTIVITE DE TYPE ANTIARTHRITIQUE CHEZ LE RAT

On utilise des groupes de 5 rats Lewis male ou femelle d'un poids de 130 à 150 g. Une suspension de 0,3 mg de Mycobactérium tuberculosis tués dans 0,1 cm³ d'huile minérale (Adjuvant de Freund complet, CFA) est administrée dans la région subplantaire de la patte arrière droite le Jour 1. Les volumes des pattes arrières sont mesurés par déplacement d'eau les Jours 0, 1, 5, 14 et 18. Les rats sont pesés aux jours 0 et 18. Les produits à tester sont placés en suspension dans de la carboxyméthyl cellulose et administrés oralement pendant 5 jours consécutifs, des jours 1 à 5.
Parallèlement un groupe témoin est utilisé afin d'éliminer les artefacts résultant de la manipulation des animaux. Un groupe traité par un produit de référence permet la validation du test.

Les produits de l'invention possèdent une puissante action inhibitrice dans ce modèle ce qui les situent comme de très intéressants candidats pour le traitement de l'arthrite.

### EXEMPLE H : COMPOSITION PHARMACEUTIQUE DESTINEE AU TRAITEMENT DES TROUBLES DU SYSTEME NERVEUX CENTRAL

Comprimés dosés à 0,1 mg de 3-méthyl 6-[2-(4-benzylpipéridin-1-yl) éthyl]benzothiazolinone

Formule pour 10 000 comprimés :
- 3-méthyl 6-[2-(4-benzylpipéridin-1-yl)éthyl]benzothiazolinone 1 g
- Amidon de blé 75 g
- Amidon de maïs 75 g
- Lactose 325g
- Stéarate de magnésium 10 g
- Silice 5 g
- Hydroxypropyl cellulose 10 g

### EXEMPLE I : COMPOSITION PHARMACEUTIQUE DESTINEE AU TRAITEMENT DES TROUBLES INFLAMMATOIRES D'ORIGINE IMMUNITAIRE

Comprimés dosés à 10 mg de 3-méthyl 6-{4-[N-(2-pipéridin-1-yl-éthyl) N-méthylamino]butyl}benzothiazolinone. Formule pour 10 000 comprimés.
- 3-méthyl 6-{4-[N-(2-pipéridin-1-yl-éthyl) N-méthylamino]butyl}benzothiazolinone 100g
- Amidon de blé 275 g
- Amidon de maïs 275 g
- Lactose 825 g
- Stéarate de magnésium 10 g
- Silice 5 g
- Hydroxypropyl cellulose 10 g

## Revendications

1. Composés de formule (I) : 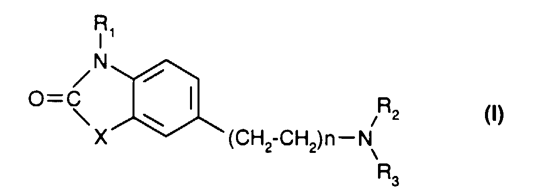 dans laquelle :
- R₁ représente un hydrogène ou un alkyle,
- n représente 1 ou 2,
- X représente un oxygène ou un soufre,
- R₂ représente un hydrogène ou un alkyle,
- et R₃ représente le groupement -(CH₂)ₘ-R₄ dans lequel :
m représente 1, 2, 3 ou 4,
et R₄ représente un groupement cycloalkylamino, dicycloalkylamino, N-cycloalkyl-N-alkylamino, ou un radical hétérocyclique de formule : 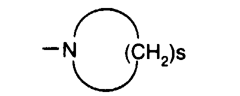 (avec s nombre entier compris entre 4 et 8 inclusivement), non substitué ou substitué par un groupement aryle, arylalkyle, aryle substitué, arylalkyl substitué,
- ou bien R₂ forme avec R₃ et l'atome d'azote qui les porte un groupement : 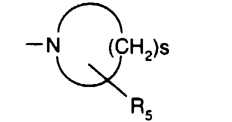 (avec s nombre entier compris entre 4 et 8 inclusivement) lequel groupement est substitué par un substituant R₅ choisi parmi aryle, arylalkyle, aryle substitué, et arylalkyle substitué,
- le terme "substitué" affectant les radicaux "aryle" et "arylalkyle" signifiant que ces groupements sont substitués par un, ou plusieurs groupements choisis parmi halogène, alkyle, hydroxyle, alkoxy, et trifluorométhyle,
- le terme "alkyle" et "alkoxy" désignent des groupements linéaires ou ramifiés contenant de 1 à 6 atomes de carbone,
- le terme "aryle" représente un groupement phényle ou naphtyle,
- le terme "cycloalkyle" désigne un groupement de 3 à 9 atomes de carbone,
leurs isomères optiques et leurs sels d'addition à un acide pharmaceutiquement acceptable et, lorsque R₁ représente un atome d'hydrogène, à une base pharmaceutiquement acceptable.

2. Composé selon la revendication 1 qui est la 3-méthyl 6-{2-[N-(2-hydroazépin-1-yléthyl)-N-méthylamino]éthyl}benzothiazolinone et ses sels d'addition à un acide pharmaceutiquement acceptable.

3. Composé selon la revendication 1 qui est la 3-méthyl 6-[2-(4-phénylpipéridine-1-yl)éthyl]benzothiazolin-2-one et ses sels d'addition à un acide pharmaceutiquement acceptable.

4. Composé selon la revendication 1 qui est la 3-méthyl 6-[2-(4-benzylpipéridin-1-yl)éthyl]benzothiazolinone et ses sels d'addition à un acide pharmaceutiquement acceptable.

5. Procédé de préparation des composés de formule (I) selon la revendication 1 caractérisé en ce que on introduit dans une solution appropriée un composé de formule (II) 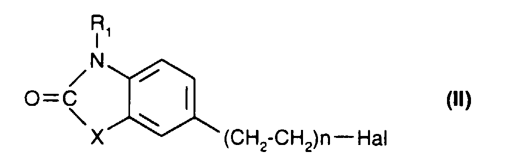 dans laquelle X, R₁ et n sont tels que définis dans la revendication 1 et Hal est un atome d'halogène, que l'on condense
- soit avec un composé de formule (III) : 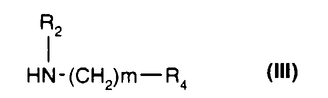 dans laquelle R₂, R₄ et m sont tels que définis dans la revendication 1 pour obtenir un composé de formule (Ia) : 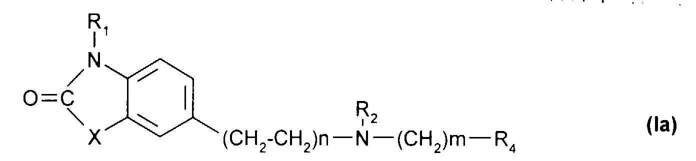 dans laquelle X, R₁, R₂, R₄, n et m sont tels que définis précédemment,
- soit avec un composé de formule (IV) : 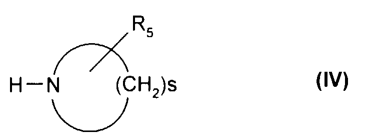 dans laquelle R₅ et s sont tels que définis dans la revendication 1,
pour obtenir un composé de formule (Ib) : 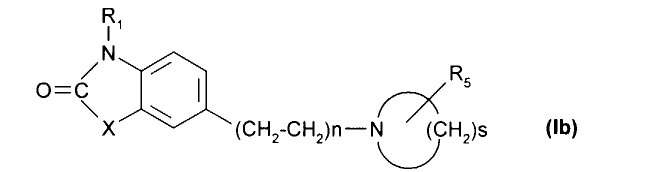 dans laquelle X, R₁, R₅ et s sont tels que définis précédemment,
composés de formule (I) selon la revendication 1 qui peuvent être, si on le désire,
- purifiés suivant une ou plusieurs méthodes de purification choisies parmi la cristallisation, la chromatographie sur gel de silice, l'extraction, la filtration et le passage sur charbon ou résine,
- séparés, le cas échéant en leurs éventuels isomères optiques,
- ou salifiés par un acide ou une base pharmaceutiquement acceptable.

6. Procédé de préparation des composés de formule (la) tels que définis dans la revendication 5, caractérisé en ce que un composé de formule (V) : 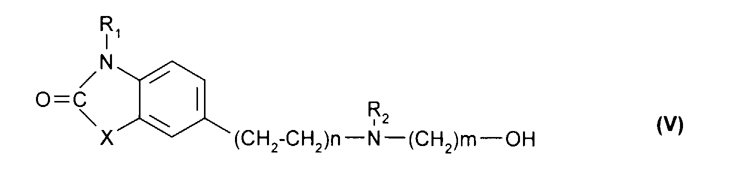 dans laquelle X, R₁, R₂, m et n sont tels que définis dans la revendicaiton 1, est traité successivement par un ou plusieurs agents d'halogénation pour conduire à un composé de formule (VI) : 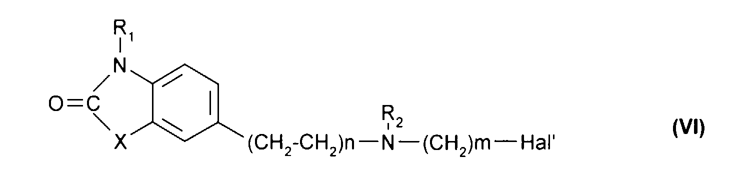 dans laquelle X, R₁, R₂, m et n sont tels que définis précédemment et Hal' représente un atome d'halogène, qui est mis en réaction avec un composé de formule (VII) :
H-R₄ **(VII)**
dans laquelle R₄ est tel que défini dans la revendication 1,
pour obtenir un composé de formule (la) tel que défini précédemment,
composés de formule (la) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant en leurs éventuels isomères optiques,
- ou salifiés par un acide ou une base pharmaceutiquement acceptable.

7. Procédé de préparation des composés de formule (la) tels que définis dans la revendication 5,
caractérisé en ce au'on condense un composé de formule (VIII) : 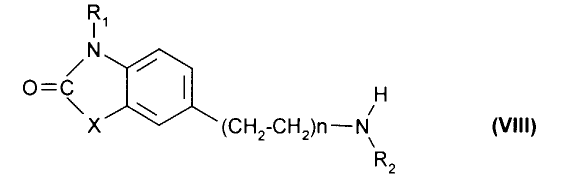 dans laquelle R₁, R₂, X et n sont tels que définis dans la revendication 1, avec un composé de formule (IX) :
Hal"-(CH₂)m-R₄ **(IX)**
dans laquelle R₄ et m sont tels que définis dans la revendication 1 et Hal" représente un atome d'halogène,
pour obtenir un composé de formule (la) tel que défini précédemment,
composés de formule (Ia) qui peuvent être, si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant en leurs éventuels isomères optiques,
- ou salifiés par un acide ou une base pharmaceutiquement acceptable.

8. Composés de formule (V) tels que définis dans la revendication 6 : 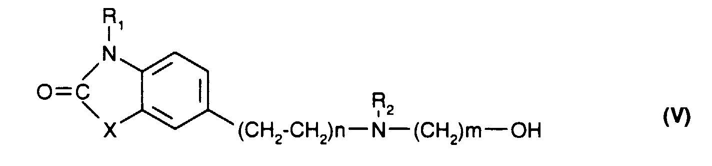 dans laquelle X, R₁, R₂, m et n sont tels que définis dans la revendication 1,
et composés de formule (VI) tels que définis dans la revendication 6 : 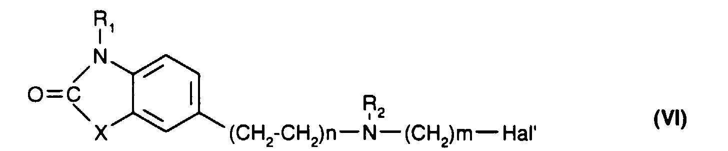 dans laquelle X, R₁, R₂, m et n sont tels que définis dans la revendication 1 et Hal' représente un atome d'halogène,
utiles comme intermédiaires de synthèse des composés de formule (I) selon la revendication 1.

9. Compositions pharmaceutiques contenant les composés de formule (I) ou le cas échéant un de leurs sels d'addition à un acide ou à une base pharmaceutiquement acceptable en combinaison avec un ou plusieurs excipients.

10. Compositions selon la revendication 9 utiles dans la prévention et le traitement des pathologies liées aux récepteurs sigma.

11. Compositions selon l'une des revendications 9 ou 10 utiles pour la prévention et le traitement des troubles psychotiques.

12. Compositions selon l'une des revendications 9 ou 10 utiles pour la prévention et le traitement de l'arthrithe aiguë ou chronique.

## Patentansprüche

1. Verbindungen der Formel (I): 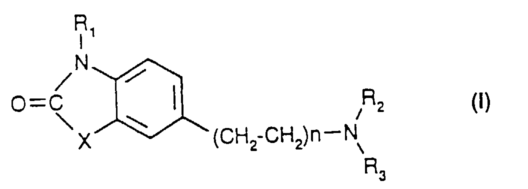 in der:
- R₁ Wasserstoff oder eine Alkylgruppe,
- n 1 oder 2,
- X Sauerstoff oder Schwefel,
- R₂ Wasserstoff oder eine Alkylgruppe und
- R₃ die Gruppe -(CH₂)ₘ-R₄ bedeuten, worin:
m 1, 2, 3 oder 4 darstellt und
R₄ eine Cycloalkylamino-, Dicycloalkylamino-, N-Cycloalkyl-N-alkylamino- oder heterocyclische Gruppe der Formel: 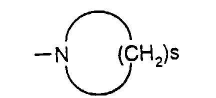 (worin s eine ganze Zahl mit einem Wert zwischen 4 und 8 einschließlich bedeutet), die nichtsubstituiert oder durch eine Arylgruppe, Arylalkylgruppe, substituierte Arylgruppe oder substituierte Arylalkylgruppe substituiert ist,
- oder R₂ zusammen mit R₃ und dem sie tragenden Stickstoffatom eine Gruppe: 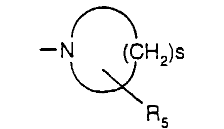 (worin s eine ganze Zahl mit einem Wert zwischen 4 und 8 einschließlich darstellt) bildet, welche Gruppe durch einen Substituenten R₅ ausgewählt aus Aryl, Arylalkyl, substituiertem Aryl und substituierten Arylalkyl substituiert ist,
- wobei der Begriff "substituiert" unter Bezug auf die "Aryl"- und "Arylalkyl"-gruppen bedeutet, daß diese Gruppen durch eine oder mehrere Gruppen ausgewählt aus Halogen, Alkyl, Hydroxyl, Alkoxy und Trifluormethyl substituiert sind,
- die Begriffe "Alkyl" und "Alkoxy" für geradkettige oder verzweigte Gruppen, die 1 bis 6 Kohlenstoffatome enthalten, stehen,
- der Begriff "Aryl" für eine Phenyl- oder Naphthylgruppe steht, und
- der Begriff "Cycloalkyl" für eine Gruppe mit 3 bis 9 Kohlenstoffatomen steht, deren optische Isomere und deren Additionssalze mit einer pharmazeutisch annehmbaren Säure und, wenn R₁ ein Wasserstoffatom darstellt, einer pharmazeutisch annehmbaren Base.

2. Verbindung nach Anspruch 1, nämlich 3-Methyl-6-{2-[N-(2-Hydroazepin-1-yl-ethyl)-N-methylamino]-ethyl}-benzothiazolinon und dessen Additionssalze mit einer pharmazeutisch annehmbaren Säure.

3. Verbindung nach Anspruch 1, nämlich 3-Methyl-6-[2-(4-Phenylpiperidin-1-yl)-ethyl]-benzothiazolin-2-on und dessen Additionssalze mit einer pharmazeutisch annehmbaren Säure.

4. Verbindung nach Anspruch 1, nämlich 3-Methyl-6-[2-(4-Benzylpiperidin-1-yl)-ethyl]-benzothiazolinon und dessen Additionssalze mit einer pharmazeutisch annehmbaren Säure.

5. Verfahren zur Herstellung der Verbindungen der Formel (I) nach Anspruch 1, dadurch gekennzeichnet, daß man in eine geeignete Lösung eine Verbindung der Formel (II) 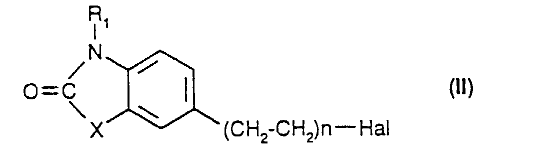 in der X, R₁ und n die in Anspruch 1 angegebenen Bedeutungen besitzen und Hal für ein Halogenatom steht, einbringt, welche man
- entweder mit einer Verbindung der Formel (III) kondensiert: 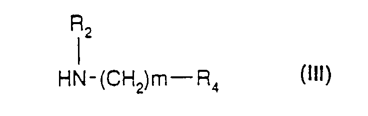 in der R₂, R₄ und m die in Anspruch 1 angegebenen Bedeutungen besitzen, zur Bildung einer Verbindung der Formel (Ia): 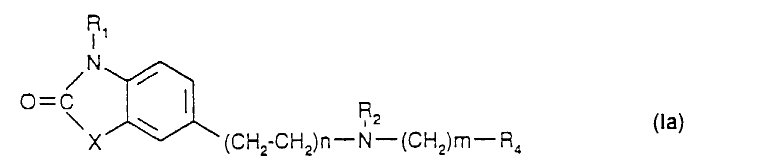 in der X, R₁, R₂, R₄, n und m die oben angegebenen Bedeutungen besitzen,
- oder mit einer Verbindung der Formel (IV) kondensiert: 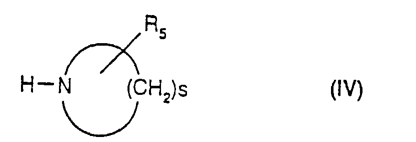 in der R₅ und s die in Anspruch 1 angegebenen Bedeutungen besitzen,
zur Bildung einer Verbindung der Formel (Ib): 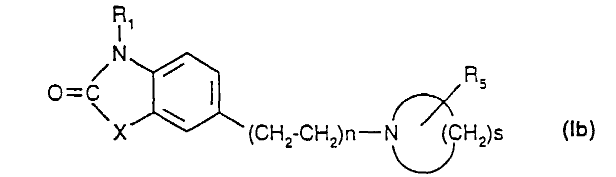 in der X, R1, R₅ und s die oben angegebenen Bedeutungen besitzen,
welche Verbindungen der Formel (I) nach Anspruch 1 gewünschtenfalls
- mit Hilfe einer oder mehrerer Reinigungsmethoden ausgewählt aus Kristallisation, Chromatographie über Kieselgel, Extraktion, Filtration und Überführung über Aktivkohle oder ein Harz gereinigt werden können,
- gegebenenfalls in ihre eventuellen optischen Isomeren aufgetrennt werden können,
- oder mit einer pharmazeutisch annehmbaren Säure oder Base in ihre Salze überführt werden können.

6. Verfahren zur Herstellung der Verbindungen der Formel (Ia), wie sie in Anspruch 5 definiert ist, dadurch gekennzeichnet, daß man eine Verbindung der Formel (V): 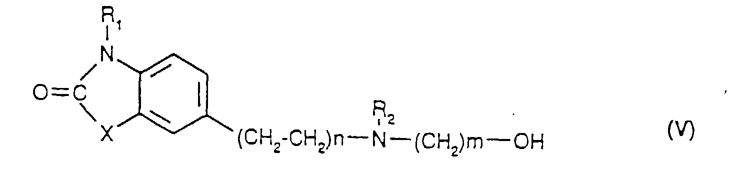 in der X, R₁, R₂, m und n die in Anspruch 1 angegebenen Bedeutungen besitzen, nacheinander mit einem oder mehreren Halogenierungsmitteln behandelt zur Bildung einer Verbindung der Formel (VI): 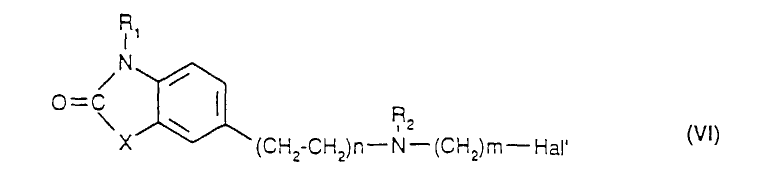 in der X, R₁, R₂, m und n die oben angegebenen Bedeutungen besitzen und Hal' ein Halogenatom darstellt, welche mit einer Verbindung der Formel (VII):
H - R₄ (VII)
in der R₄ die in Anspruch 1 angegebenen Beudeutungen besitzt, umgesetzt wird zur Bildung einer Verbindung der oben definierten Formel (Ia),
welche Verbindungen der Formel (Ia) gewünschtenfalls
- mit Hilfe einer oder mehrerer Reinigungsmethoden ausgewählt aus Kristallisation, Chromatographie über Kieselgel, Extraktion, Filtration und Überführung über Aktivkohle oder ein Harz gereinigt werden können,
- gegebenenfalls in ihre eventuellen optischen Isomeren aufgetrennt werden können,
- oder mit einer pharmazeutisch annehmbaren Säure oder Base in ihre Salze überführt werden können.

7. Verfahren zur Herstellung der Verbindungen der Formel (Ia), wie sie in Anspruch 5 definiert ist,
dadurch gekennzeichnet, daß man eine Verbindung der Formel (VIII): 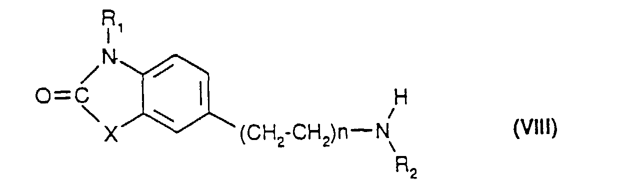 in der R₁, R₂, X und n die in Anspruch 1 angegebenen Bedeutungen besitzen, mit einer Verbindung der Formel (IX):
Hal" - (CH₂)ₘ - R₄ (IX)
in der R₄ und m die in Anspruch 1 angegebenen Bedeutungen besitzen und Hal" ein Halogenatom darstellt, kondensiert,
zur Bildung einer Verbindung der oben definierten Formel (Ia),
welche Verbindungen der Formel (Ia) gewünschtenfalls
- mit Hilfe einer oder mehrerer Reinigungsmethoden ausgewählt aus Kristallisation, Chromatographie über Kieselgel, Extraktion, Filtration und Überführung über Aktivkohle oder ein Harz gereinigt werden können,
- gegebenenfalls in ihre eventuellen optischen Isomeren aufgetrennt werden können,
- oder mit einer pharmazeutisch annehmbaren Säure oder Base in ihre Salze überführt werden können.

8. Verbindungen der Formel (V), wie sie in Anspruch 6 definiert sind: 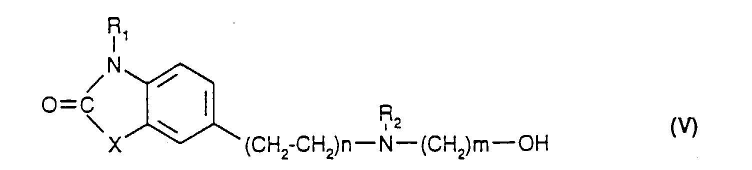 in der X, R₁, R₂, m und n die in Anspruch 1 angegebenen Bedeutungen besitzen, und Verbindungen der Formel (VI), wie sie in Anspruch 6 definiert sind: 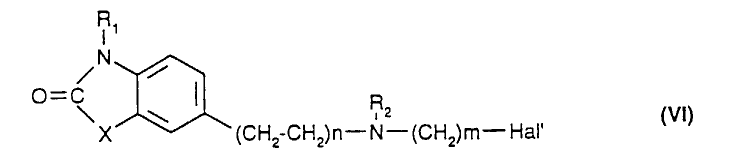 in der X, R₁, R₂, m und n die in Anspruch 1 angegebenen Bedeutungen besitzen und Hal' ein Halogenatom darstellt,
als Zwischenprodukte für die Synthese der Verbindungen der Formel (I) nach Anspruch 1.

9. Pharmazeutische Zubereitungen enthaltend die Verbindungen der Formel (I) oder gegebenenfalls eines ihrer Additionssalze mit einer pharmazeutisch annehmbaren Säure oder Base in Kombination mit einem oder mehreren Trägermaterialien.

10. Zubereitungen nach Anspruch 9 zur Vorbeugung und zur Behandlung von mit Sigma-Rezeptoren verknüpften pathologischen Zuständen.

11. Zubereitungen nach einem der Ansprüche 9 oder 10 zur Vorbeugung und zur Behandlung von psychotischen Störungen.

12. Zubereitungen nach einem der Ansprüche 9 oder 10 zur Vorbeugung und zur Behandlung von akuter oder chronischer Arthritis.

## Claims

1. Compounds of formula (I): 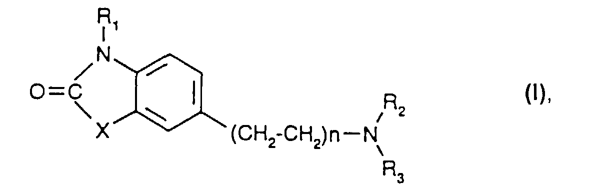 wherein :
- R₁ represents a hydrogen atom or an alkyl group,
- n represents 1 or 2,
- X represents an oxygen or sulphur atom,
- R₂ represents a hydrogen atom or an alkyl group,
- and R₃ represents the group -(CH₂)ₘ-R₄ wherein :
m represents 1, 2, 3 or 4,
and R₄ represents a cycloalkylamino, dicycloalkylamino or N-cycloalkyl-N-alkylamino group, or a heterocyclic radical of formula : 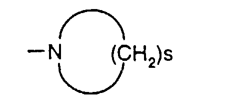 (s being an integer from 4 to 8 inclusive), unsubtituted or substituted by an aryl, arylalkyl, substituted aryl or substituted arylalkyl group,
- or R₂, together with R₃ and the nitrogen atom carrying them, forms a group : 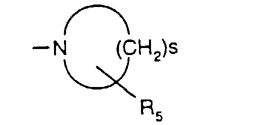 (s being an integer from 4 to 8 inclusive), which group is substituted by a substituent R₅ selected from aryl, arylalkyl, substituted aryl and substituted arylalkyl,
- the term "substituted" associated with the "aryl" and "arylalkyl" radicals indicating that the groups in question are substituted by one or more groups selected from halogen, alkyl, hydroxyl, alkoxy and trifluoromethyl,
- the terms "alkyl" and "alkoxy" indicating linear or branched groups containing from 1 to 6 carbon atoms,
- the term "aryl" denoting a phenyl or naphthyl group,
- the term "cycloalkyl" indicating a group having from 3 to 9 carbon atoms,
their optical isomers and addition salts thereof with a pharmaceutically acceptable acid or, when R₁ represents a hydrogen atom, with a pharmaceutically acceptable base.

2. Compound according to claim 1 that is 3-methyl-6-{2-[N-(2-hydroazepin-1-yl-ethyl)-N-methylamino]ethyl}benzothiazolinone and addition salts thereof with a pharmaceutically acceptable acid.

3. Compound according to claim 1 that is 3-methyl-6-[2-(4-phenylpiperidin-1-yl)ethyl]-benzothiazolin-2-one and addition salts thereof with a pharmaceutically acceptable acid.

4. Compound according to claim 1 that is 3-methyl-6-[2-(4-benzylpiperidin-1-yl)ethyl]-benzothiazolinone and addition salts thereof with a pharmaceutically acceptable acid.

5. Process for the preparation of compounds of formula (I) according to claim 1, characterised in that there is incorporated in an appropriate solution a compound of formula (II): 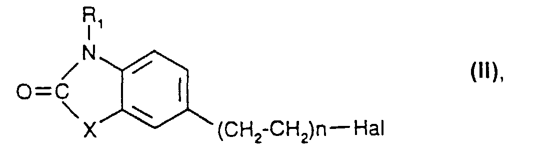 wherein X, R₁ and n are as defined in claim 1 and Hal is a halogen atom, which is condensed with
- either a compound of formula (III): 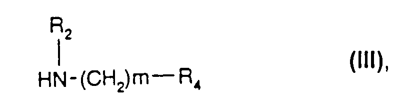 wherein R₂, R₄ and m are as defined in claim 1, to obtain a compound of formula (Ia): 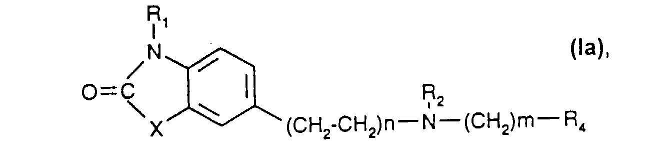 wherein X, R₁, R₂, R₄, n and m are as defined hereinbefore,
- or with a compound of formula (IV): 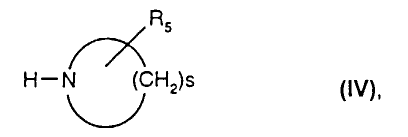 wherein R₅ and s are as defined in claim 1,
to obtain a compound of formula (Ib): 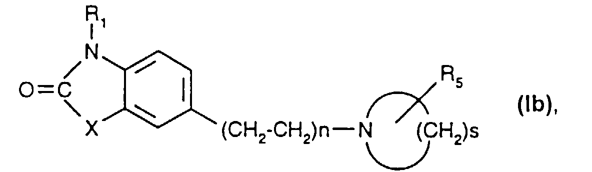 wherein X, R₁, R₅ and s are as defined hereinbefore,
which compounds of formula (I) according to claim 1 may be, if desired,
- purified by one or more purification methods selected from crystallisation, chromatography on silica gel, extraction, filtration, and passage over carbon or resin,
- separated, where appropriate, into their possible optical isomers,
- or converted into salts with a pharmaceutically acceptable acid or base.

6. Process for the preparation of compounds of formula (la) as defined in claim 1, characterised in that a compound of formula (V): 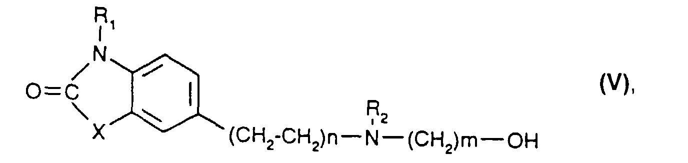 wherein X, R₁, R₂, m and n are as defined in claim 1,
is treated successively with one or more halogenating agents to yield a compound of formula (VI): 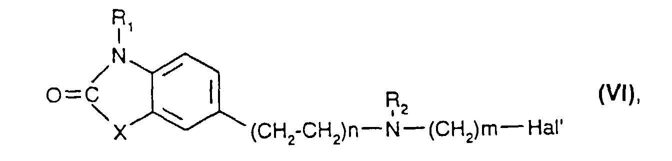 wherein X, R₁, R₂, m and n are as defined hereinbefore and Hal' represents a halogen atom, which is reacted with a compound of formula (VII):
H - R₄ **(VII),**
wherein R₄ is as defined in claim 1,
to obtain a compound of formula (la) as defined hereinbefore,
which compounds of formula (la) may be, if desired,
- purified by one or more purification methods selected from crystallisation, chromatography on silica gel, extraction, filtration, and passage over carbon or resin,
- separated, where appropriate, into their possible optical isomers,
- or converted into salts with a pharmaceutically acceptable acid or base.

7. Process for the preparation of compounds of formula (la) as defined in claim 5, characterised in that a compound of formula (VIII): 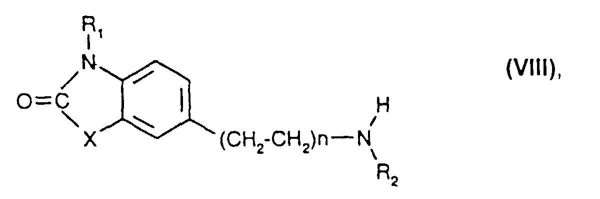 wherein R₁, R₂, X and n are as defined in claim 1, is condensed with a compound of formula (IX):
Hal" - (CH₂)m - R₄ **(IX),**
wherein R₄ and m are as defined in claim 1 and Hal" represents a halogen atom, to obtain a compound of formula (la) as defined hereinbefore,
which compounds of formula (la) may be, if desired,
- purified by one or more purification methods selected from crystallisation, chromatography on silica gel, extraction, filtration, and passage over carbon or resin,
- separated, where appropriate, into their possible optical isomers,
- or converted into salts with a pharmaceutically acceptable acid or base.

8. Compounds of formula (V) as defined in claim 6 : 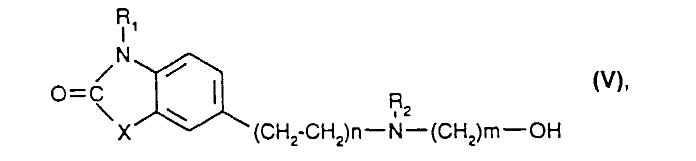 wherein X, R₁, R₂, m and n are as defined in claim 1,
and compounds of formula (VI) as defined in claim 6 : 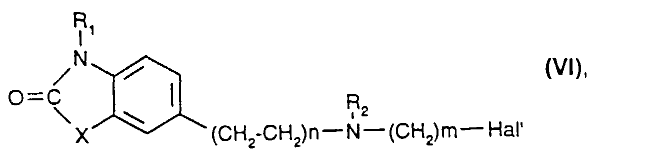 wherein X, R₁, R₂, m and n are as defined in claim 1 and Hal' represents a halogen atom,
for use as synthesis intermediates for compounds of formula (I) according to claim 1.

9. Pharmaceutical compositions comprising compounds of formula (I) or, where appropriate, an addition salt thereof with a pharmaceutically acceptable acid or base, in combination with one or more excipients.

10. Compositions according to claim 9 for use in the prevention and treatment of pathologies associated with sigma receptors.

11. Compositions according to claim 9 or claim 10 for use in the prevention and treatment of psychotic disorders.

12. Compositions according to claim 9 or claim 10 for use in the prevention and treatment of acute or chronic arthritis.
